# EUROPEAN PATENT SPECIFICATION

(11) **EP 2 422 732 B1**
(45) Date of publication and mention of the grant of the patent: **25.11.2015**
(21) Application number: 10766959.0
(22) Date of filing: 07.04.2010
(51) Int. Cl.: A61B 18/00, A61B 18/04, H01H 25/06, A61B 17/32, A61B 18/14, H01H 13/06, H01H 13/08

(54) **SWITCH STRUCTURE AND SURGERY APPARATUS**
SCHALTSTRUKTUR UND CHIRURGISCHES GERÄT DAMIT
STRUCTURE DE COMMUTATION ET APPAREIL CHIRURGICAL

(30) Priority: 22.04.2009 US 427824
(43) Date of publication of application: 29.02.2012
(73) Proprietor: Olympus Corporation, Shibuya-ku Tokyo (JP)
(72) Inventor: YACHI, Chie, Tokyo 192-8512 (JP); FUJII, Yoshitaka, Tokyo 192-8512 (JP)
(74) Representative: Schicker, Silvia
(86) International application number: PCT/JP2010/056335
(87) International publication number: WO 2010/122901

(56) References cited:
- JP-A- 10 283 885
- JP-A- 2005 243 320
- JP-A- 2007 317 392
- JP-A- 2008 168 138
- US-A- 6 124 555
- US-A1- 2008 091 072
- US-A1- 2009 088 667

## Description

### Technical Field

The present invention relates to an operation device configured to control driving of an apparatus such as a surgical apparatus and a surgical apparatus including the operation device.

### Background Art

In general, there is a device as a surgical apparatus disclosed in, e.g., Patent Literature 1. A main body operation portion of this device is provided with a switch configured to control driving of the device. The switch includes a pressure-receiving portion configured to be pressed by a user, an activation rod configured to be activated when the pressure-receiving portion is pressed, and a switch contact point. The pressure-receiving portion is provided at one end side of the activation rod, and the switch contact point is provided at the other end side of the activation rod. During operation in which a user pushes the pressure-receiving portion, the activation rod moves in an axial direction and presses the switch contact point. Thereby, the driving of the device is controlled.

US 2008/0091072 discloses a switch element including a pad and a board. When an upper end face of the pad is not pressed, the pad is in a neutral position that is not inclined. In the neutral position, the pad is urged by a folded web. As a result, a gap is formed between the board and a bottom end face of a protrusion provided on the pad. Thus, the bottom end face of the protrusion is not in contact with the sensors on the board. By pressing a left slanted surface of an upper end face, the pad is inclined from the neutral position toward left, and a left portion of the bottom end surface is brought into contact with a sensor on the left. On the other hand, by pressing a right slanted surface of the upper end face, the pad is inclined from the neutral position toward right, and the right portion of the bottom end surface is brought into contact with a sensor on the right.

US 2009/0088667 A1 discloses a surgical operating apparatus. US 6,124,555 discloses a metallic operating lever which can be tilted: the shape of the operating lever is complementary to the shape of a driver made of synthetic resin.

### Citation List

### Patent Literature

Patent Literature 1: Jpn. Pat. Appln. KOKAI Publication No. 2002-291745

### Summary of Invention

### Technical Problem

In the device disclosed in the above Patent Literature 1, the activation rod moves straight only in the axial direction during operation in which the user pushes the pressure-receiving portion of the switch. Accordingly, when the user operates the switch, the user has to push the pressure-receiving portion at the central position of the pressure-receiving portion to move the activation rod straight in the axial direction. When the user pushes the pressure-receiving portion at a position away from the central position of the pressure-receiving portion, a load is applied obliquely with respect to the axial direction of the activation rod. In this case, an excessive load is applied to the entire switch, which may deform component parts, and there is a possibility that the driving of the device is not correctly controlled. As described above, when the user operates the switch, unless the user moves the activation rod straight in the axial direction at the central position of the pressure-receiving portion, there is a possibility that the driving of the device is not correctly controlled, which reduces the ease of operation when the user operates the switch.

The present invention is made in view of the above problem, and it is an object of the present invention to provide an operation device and a surgical apparatus, wherein even when a user presses a pressure-receiving portion at a position displaced in any given direction from a central position of the pressure-receiving portion during operation of a switch, a switch contact point can be pressed and operated stably.

### Solution to Problem

The present invention is defined in claim 1.

According to one aspect of the invention, a switch structure configured to control driving of an apparatus, includes that an operation portion housing configured to be held by a user; a switch contact point which is provided in the operation portion housing; a switch operation portion which includes a pressure-receiving portion configured to be pressed by the user, the switch operation portion being configured to operate the switch contact point between an open state and a closed state; and a support portion configured to support the pressure-receiving portion in such a manner that the pressure-receiving portion can incline and move about an inclination movement center in the operation portion housing with respect to the operation portion housing, the support portion being configured to support the pressure-receiving portion in such a manner that the user can press the pressure-receiving portion in a direction where the inclination movement center of the pressure-receiving portion is arranged in a state in which the pressure-receiving portion is inclined and moved and in a neutral position state in which the pressure-receiving portion is not inclined and moved.

The operation device further includes an urging member configured to maintain the switch contact point in the open state during a non-pressed operation of the pressure-receiving portion in which the switch operation portion is maintained at a position where the switch contact point is maintained in the open state by the support portion; and a switch substrate which includes a transmission section configured to transmit a signal of driving the apparatus during a close operation of the switch contact point in which the pressure-receiving portion is pressed and the switch operation portion is maintained by the support portion in such a manner that the switch operation portion can move in a direction where the switch contact point is closed against a force given by the urging member, the switch substrate being configured to close the switch contact point when pressed by the switch operation portion.

The switch operation portion includes: a shaft section which is disposed between the pressure-receiving portion and the switch contact point; a first coupling section which is disposed at an end portion on a pressure-receiving portion side of the shaft section, the first coupling section coupling with the pressure-receiving portion; and a second coupling section which is disposed at an end portion on a switch contact point side of the shaft section, the second coupling section coupling with the operation portion housing, and the support portion includes a clearance formation section configured to form a clearance between the support portion and a first coupling section side part of the shaft section, the clearance formation section being configured to cause the pressure-receiving portion to be movable in an inclination movement directions about the inclination movement center of the pressure-receiving portion. Also, the clearance formation section includes an inclination surface whose size of cross sectional area of the clearance gradually increases from the switch contact point side to the pressure-receiving portion side.

The second coupling section includes: a first protrusion which protrudes in a first inclination movement direction of the switch operation portion that inclines from the neutral position to two directions including at least the first inclination movement direction and a second inclination movement direction, the first protrusion being configured to form a first inclination movement support point at a contact section with an inner surface of the operation portion housing when the pressure-receiving portion of the switch operation portion is inclined and moved in the second inclination movement direction; a second protrusion which protrudes in the second inclination movement direction of the switch operation portion, the second protrusion being configured to form a second inclination movement support point at a contact section with the inner surface of the operation portion housing when the pressure-receiving portion is inclined and moved in the first inclination movement direction; and a switch contact section configured to be in contact with the switch substrate, the switch contact section being configured to press the switch substrate in the direction where the switch contact point is closed in accordance with a movement in which the shaft section of the switch operation portion inclines and moves about the first inclination movement support point in the second inclination movement direction or in accordance with a movement in which the shaft section inclines and moves about the second inclination movement support point in the first inclination movement direction. Further, it is preferable that the pressure-receiving portion includes an arc-shaped pressing surface extended along two directions including the first inclination movement direction and the second inclination movement direction.

The second coupling section includes: a protrusion which protrudes in outer peripheral directions of the switch operation portion that can incline and move from the neutral position in any one of the given inclination movement directions, a part of the protrusion located on an opposite side to said any one of the inclination movement directions of the pressure-receiving portion with respect to the inclination movement center of the pressure-receiving portion being configured to form an inclination movement support point at a contact section with an inner surface of the operation portion housing when the pressure-receiving portion of the switch operation portion is inclined and moved from the neutral position to said any one of the given inclination movement directions; and a switch contact section configured to be in contact with the switch substrate, the switch contact section being configured to press the switch substrate in a direction where the switch contact point is closed in accordance with movement in which the shaft section of the switch operation portion inclines and moves about the inclination movement support point in said any one of the inclination movement directions of the pressure-receiving portion.

The switch operation portion is configured to incline and move from the neutral position in a pressed position direction that is a direction in which a position pressed by the user is located with respect to a central position of the pressure-receiving portion.

It is preferable that the pressure-receiving portion of the switch operation portion can incline and move within a movable range of a finger of the user.

It is preferable that the operation device includes at least one or more switch operation portion/portions, wherein the operation portion housing includes a protuberance section which is disposed between the switch operation portions and on which a finger of the user can be placed during operation.

According to one another aspect of the invention, a surgical apparatus includes that a hand piece which includes an operation portion housing configured to be held by a user; and a switch configured to control driving of the apparatus, wherein the switch includes: a switch contact point which is arranged in the operation portion housing; a switch operation portion which includes a pressure-receiving portion configured to be pressed by the user, the switch operation portion being configured to operate the switch contact point between an open state and a closed state; and a support portion configured to support the pressure-receiving portion in such a manner that the pressure-receiving portion can incline and move about an inclination movement center in the operation portion housing with respect to the operation portion housing, the support portion being configured to support the pressure-receiving portion in such a manner that the user can press the pressure-receiving portion in a direction where the inclination movement center of the pressure-receiving portion is arranged in a state in which the pressure-receiving portion is inclined and moved and in a neutral position state in which the pressure-receiving portion is not inclined and moved.

The switch of the surgical apparatus includes: an urging member configured to maintain the switch contact point in the open state during a non-pressed operation of the pressure-receiving portion in which the switch operation portion is maintained at a position where the switch contact point is maintained in the open state by the support portion; and a switch substrate which includes a transmission section configured to transmit a signal of driving the apparatus during a close operation of the switch contact point in which the pressure-receiving portion is pressed and the switch operation portion is maintained by the support portion in such a manner that the switch operation portion can move in a direction where the switch contact point is closed against a force given by the urging member, the switch substrate being configured to close the switch contact point when pressed by the switch operation portion.

It is preferable that the surgical apparatus which is an ultrasonic surgical apparatus further includes: an electrical connection section configured to electrically connect the hand piece and a generator configured to generate drive energy of driving the hand piece; an ultrasonic vibrator which is provided in the hand piece and is electrically connected to the generator; and an ultrasonic probe which is connected to the ultrasonic vibrator, ultrasonic vibration from the ultrasonic vibrator is configured to be transmitted to the ultrasonic probe. Also, it is preferable that the surgical apparatus further includes: a sheath which covers the ultrasonic probe; and a jaw which is attached to a distal end portion of the sheath in such a manner that the jaw can pivot with respect to a distal end portion of the ultrasonic probe.

It is preferable that the surgical apparatus further includes: an electrical connection section configured to electrically connect the hand piece and a generator configured to generate drive energy of driving the hand piece; and a conductive probe provided in the hand piece and electrically connected to the generator, wherein the surgical apparatus is a high-frequency surgical apparatus capable of controlling, with the switch, a high-frequency current supplied from the generator to the probe.

It is preferable that the surgical apparatus further includes: an electrical connection section configured to electrically connect the hand piece and a generator configured to generate drive energy of driving the hand piece; an ultrasonic vibrator which is provided in the hand piece and is electrically connected to the generator; and a conductive probe which is connected to the ultrasonic vibrator and electrically connected to the generator, wherein the surgical apparatus is a surgical operating apparatus capable of controlling, with the switch, a high-frequency current supplied from the generator to the probe and an ultrasonic drive current supplied from the generator to the ultrasonic vibrator.

### Advantageous Effects of Invention

According to the present invention, an operation device and a surgical apparatus can be provided, wherein even when a user presses a pressure-receiving portion at a position displaced in any given direction from a central position of the pressure-receiving portion during operation of a switch, a switch contact point can be pressed and operated stably.

### Brief description of Drawings

FIG. 1 is a perspective view illustrating a schematic configuration of an entire hand piece of a surgical operating apparatus according to a first embodiment of the present invention;
FIG. 2 is a front view of the hand piece of the surgical operating apparatus according to the first embodiment;
FIG. 3 is a side view illustrating a handle unit of the surgical operating apparatus according to the first embodiment;
FIG. 4 is a perspective view illustrating a disassembled state in which coupling portions of an assemble unit of the surgical operating apparatus according to the first embodiment are detached;
FIG. 5 is a longitudinal sectional view illustrating the hand piece of the surgical operating apparatus according to the first embodiment;
FIG. 6 is a sectional view taken along line VI-VI of FIG. 5;
FIG. 7 is a sectional view taken along line VII-VII of FIG. 6;
FIG. 8 is a sectional view taken along line VIII-VIII of FIG. 6;
FIG. 9 is a sectional view taken along line IX-IX of FIG. 6;
FIG. 10 is a sectional view taken along line X-X of FIG. 6;
FIG. 11 is a sectional view taken along line XI-XI of FIG. 5;
FIG. 12 is a top view illustrating a circuit pattern of a switch substrate of a hand switch in the hand piece of the surgical operating apparatus according to the first embodiment;
FIG. 13 is a longitudinal sectional view illustrating a pressure-receiving portion of the hand switch in the hand piece of the surgical operating apparatus according to the first embodiment in a state that the pressure-receiving portion is maintained in a neutral position;
FIG. 14 is a longitudinal sectional view illustrating the pressure-receiving portion of the hand switch in the hand piece of the surgical operating apparatus according to the first embodiment in a state that the pressure-receiving portion is pressed to a position at the left side with respect to the central position of a pressing surface;
FIG. 15 is a longitudinal sectional view illustrating the pressure-receiving portion of the hand switch in the hand piece of the surgical operating apparatus according to the first embodiment in a state that the pressure-receiving portion is pressed to a position at the right side with respect to the central position of a pressing surface;
FIG. 16 is a perspective view illustrating a surgical operating apparatus according to a second embodiment of the present invention;
FIG. 17 is a longitudinal sectional view illustrating a pressure-receiving portion of a hand switch in a hand piece of the surgical operating apparatus according to the second embodiment in a state that the pressure-receiving portion is maintained in a neutral position;
FIG. 18 is a perspective view illustrating a configuration of a switch operation portion of the hand switch in the hand piece of the surgical operating apparatus according to the second embodiment;
FIG. 19 is a side view illustrating a hand piece of a surgical operating apparatus according to a third embodiment of the present invention;
FIG. 20 is a side view illustrating a hand piece of a surgical operating apparatus according to a fourth embodiment of the present invention; and
FIG. 21 is a perspective view illustrating a hand piece of a surgical operating apparatus according to a fifth embodiment of the present invention.

### Description of Embodiments

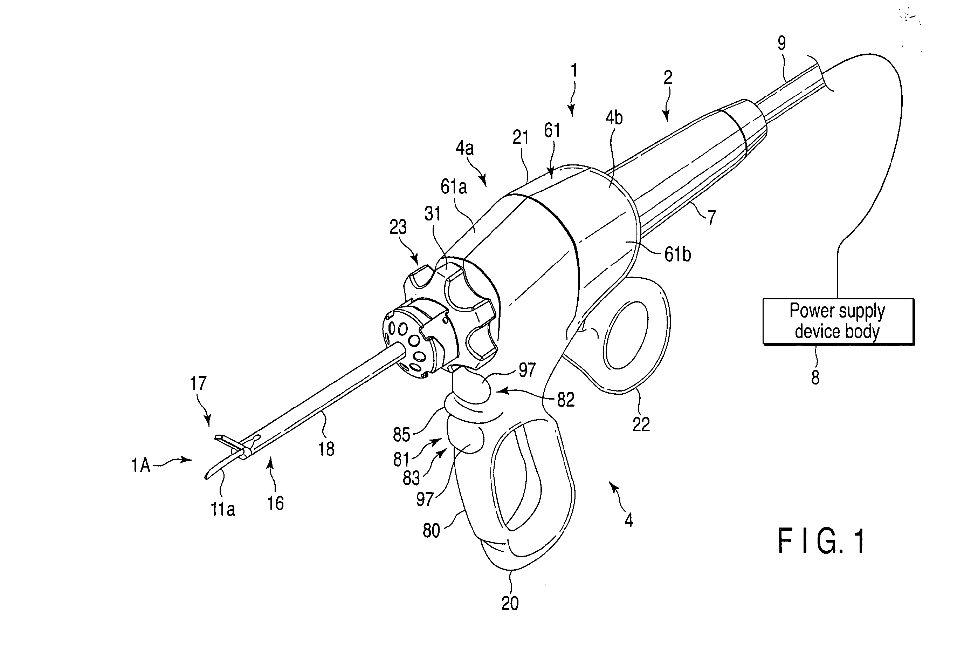

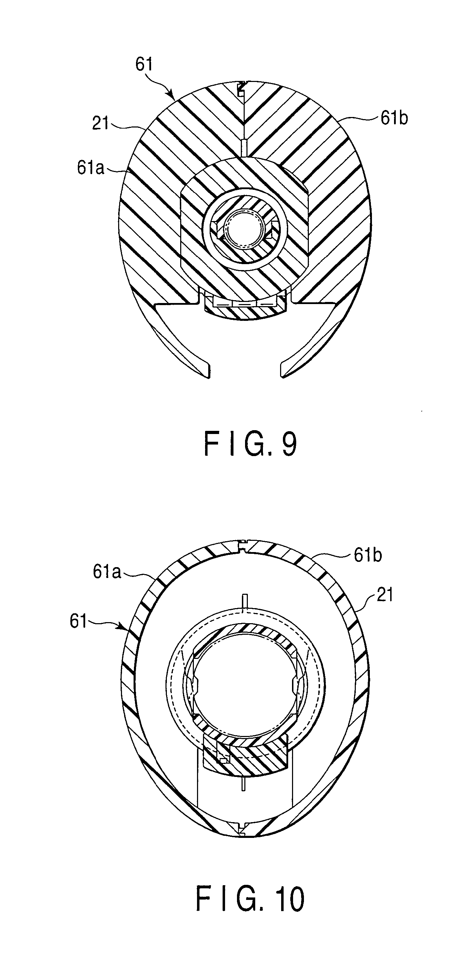

Hereinafter, the first embodiment of the present invention will be described with reference to FIGS. 1 to 15. FIG. 1 is a view illustrating a schematic configuration of an entire hand piece 1 of a surgical operating apparatus according to the present embodiment. The surgical operating apparatus according to the present embodiment is an ultrasonic coagulation-incision treatment apparatus. This ultrasonic coagulation-incision treatment apparatus can perform a treatment such as incision, resection, or coagulation of a living tissue by utilizing ultrasonic waves, as well as a treatment utilizing high-frequency waves.

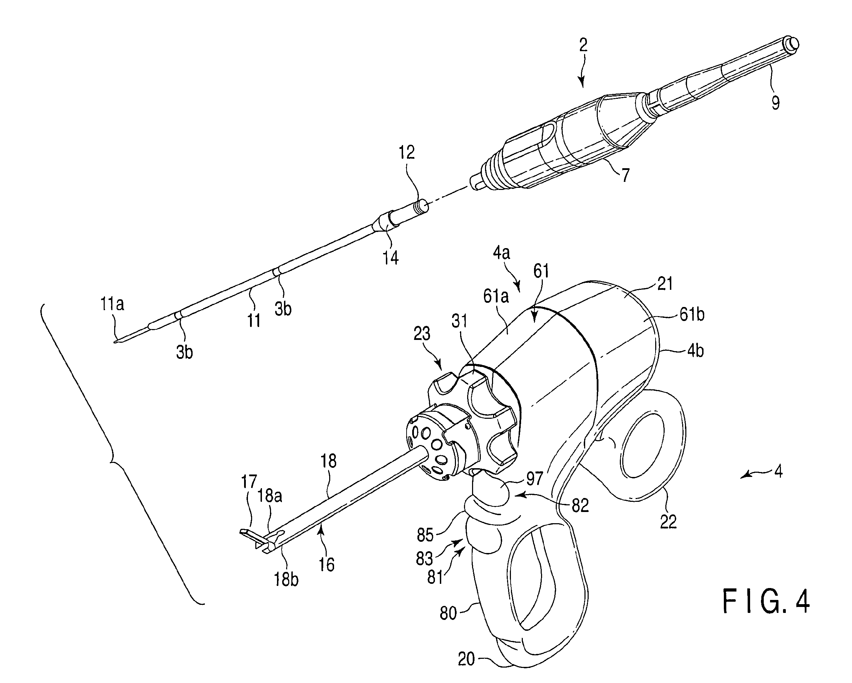

As shown in FIG. 4, the hand piece 1 includes a vibrator unit 2 and a handle unit (handle section) 4. The vibrator unit 2 and handle unit 4 are detachably coupled with each other.

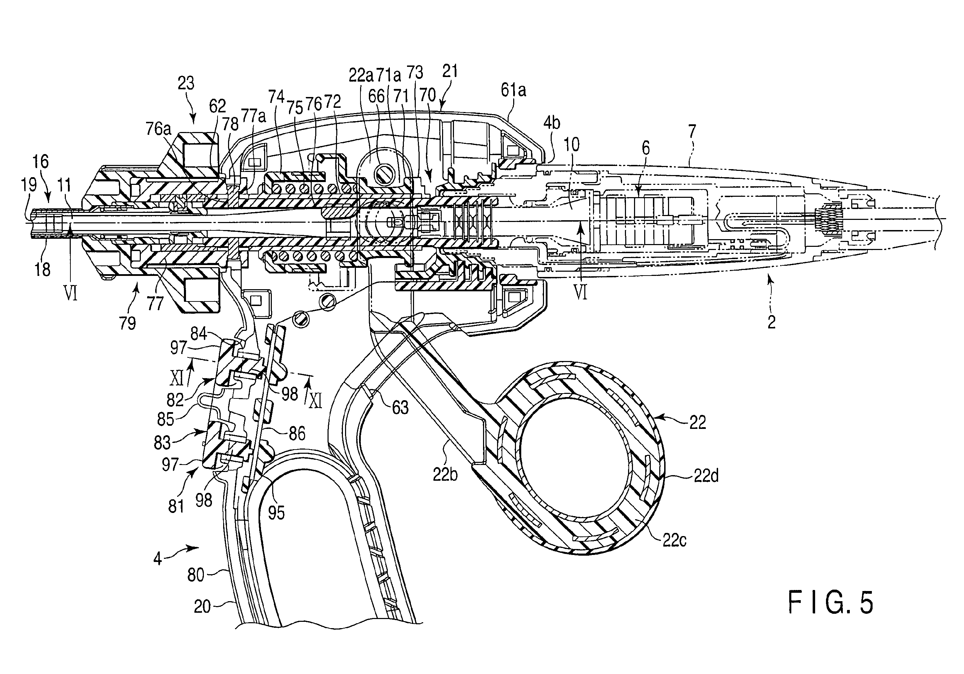

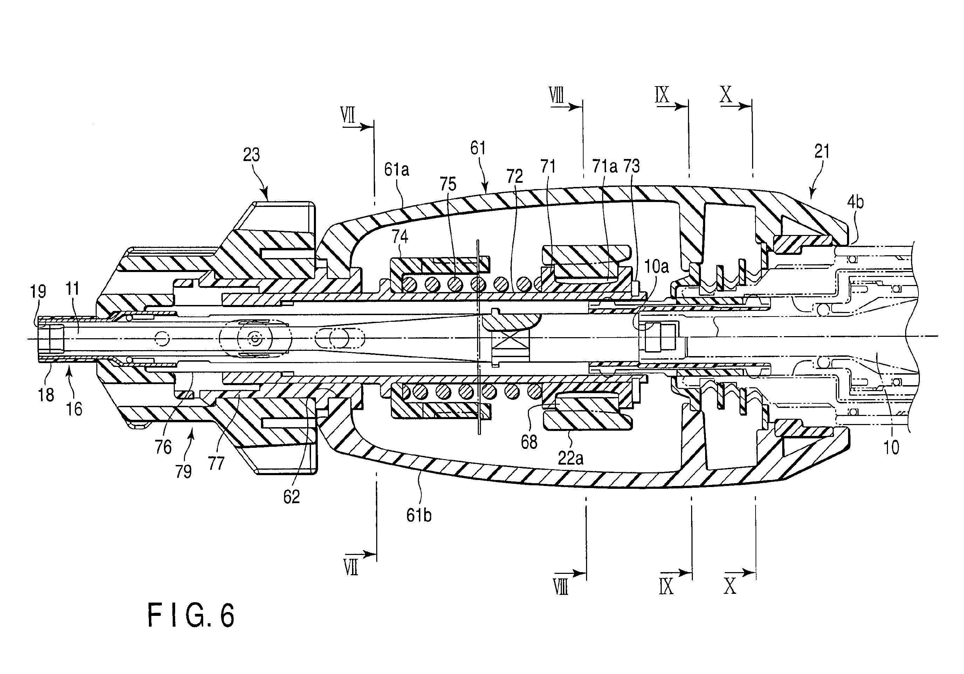

A vibrator 6 (see FIG. 5) is provided in the vibrator unit 2. The vibrator 6 generates ultrasonic vibration by a piezoelectric element which converts an electric current into ultrasonic vibration. An outside of the vibrator 6 is covered with a circular cylindrical vibrator cover 7. Further, a cable 9 configured to supply an electric current which generates ultrasonic vibration from a power supply device body 8 is extended to a proximal end side of the vibrator unit 2. As shown in FIG. 5, a proximal end portion of a horn 10, which increases amplitude of ultrasonic vibration, is coupled to a distal end portion of the ultrasonic vibrator 6 within the vibrator cover 7. A screw hole portion 10a (see FIG. 6), to which a probe is attached, is formed at a distal end portion of the horn 10.

The ultrasonic probe 11 is designed in such a manner that an overall length of the ultrasonic probe 11 is an integral multiple of a half wavelength of the ultrasonic vibration. A screw portion 12 is provided at a proximal end portion of the ultrasonic probe 11. The screw portion 12 is engaged with the screw hole portion 10a of the horn 10. The screw portion 12 of the ultrasonic probe 11 is engaged with the screw hole portion 10a of the horn 10 of the vibrator unit 2. Thereby, the ultrasonic probe 11 and the vibrator 6 are assembled. At this time, a first high-frequency electric path (not shown), through which a high-frequency current is transmitted, is formed in the connected body of the ultrasonic vibrator 6 and the ultrasonic probe 11.

A probe distal end 11a is provided at a distal end portion of the ultrasonic probe 11. The probe distal end 11a is formed in a substantially J-shaped curved form. A flange portion 14 is provided at a position of a most proximal end side node of vibration in an axial direction of the ultrasonic probe 11. Engaging recess portions each having a key groove shape are formed on the outer peripheral surface of the flange portion 14 at three positions in the circumferential direction thereof (not shown).

Further, the probe distal end 11a includes a first electrode section which is one of bipolar electrodes. In the ultrasonic probe 11, the cross-sectional area taken in perpendicular to the axial direction is reduced at several node positions of the vibration, each of which is provided on a midway in the axial direction, so that amplitude necessary for the treatment can be obtained in the treatment with the probe distal end 11a. Each of Rubber rings 3b formed of an elastic member is attached to the ultrasonic probe 11 at a corresponding position of several node positions, each of which is provided on the midway in the axial direction of the ultrasonic probe 11, of the vibration. Further, the rubber rings 3b prevent interference between the ultrasonic probe 11 and a sheath body 16 explained later.

As shown in FIG. 4, the handle unit 4 includes an elongated sheath body 16, a jaw 17 which is provided at a distal end portion of the sheath body 16, and an operation portion 4a provided to a proximal end side of the sheath body 16. A holding tube 21 in a substantially cylindrical shape is provided at the operation portion 4a of the handle unit 4. A vibrator connection portion 4b is formed at a proximal end portion of the holding tube 21.

The sheath body 16 includes an outer sheath 18 made of metal which is an external cylinder, and a drive pipe (drive member) 19 made of metal which is an internal cylinder (inner sheath). The drive pipe 19 is inserted into the outer sheath 18 so as to be movable in the axial direction. An proximal end portion of the outer sheath 18 as well as a rotation operation knob 23, explained later, are integrally attached to the distal end portion of the holding tube 21 in such a manner that the outer sheath 18 can rotate in a direction about an axis of the outer sheath 18.

A pair of right-and-left projection pieces 18a, 18b are provided at the distal end portion of the outer sheath 18 in such a manner that the projection pieces 18a, 18b protrude toward the distal end side. The proximal end part of the jaw 17 is pivotally attached to each of the projection pieces 18a, 18b through a support pin, not shown.

The jaw 17 is formed into a curved shape such as a substantially J-shape corresponding to the curved shape of the probe distal end 11a of the ultrasonic probe 11. When the ultrasonic probe 11 and the handle unit 4 are coupled to each other, the jaw 17 is arranged at a position at which the jaw 17 faces to the probe distal end 11a of the ultrasonic probe 11.

The jaw 17 includes a jaw body (not shown) made of a conductive member such as metal and a grasping member (not shown) to be attached to the jaw body. The grasping member is constituted of an electrode member used in high-frequency treatment and a pad member used in ultrasonic treatment. The electrode member constitutes a second electrode section which is the other of the bipolar electrodes. The pad member is formed of an insulator which is a resin material such as polytetrafluoroethylene and the like.

Further, a distal end portion of the drive pipe 19 is coupled with the jaw 17 via a coupling pin, not shown. The jaw body of the jaw 17 and the drive pipe 19 are electrically connected via the coupling pin.

An outer peripheral surface of the outer sheath 18 is covered with an outer coat formed of an insulating material such as resin. An insulating tube formed of an insulating material is provided to the inner peripheral surface side of the drive pipe 19. A proximal end portion of the insulating tube is extended to a proximal end side part of the sheath body 16. The drive pipe 19 and the ultrasonic probe 11 are electrically insulated by the insulating tube.

The operation portion 4a of the handle unit 4 includes a fixed handle 20 and a movable handle 22 that can pivot with respect to the fixed handle 20. The handle unit 4 includes an operation portion housing 61 formed of a resin material integrally with a holding tube 21 and a fixed handle 20. As shown in FIGS. 6 to 10, the operation portion housing 61 includes two housing components (a left side housing component 61a and a right side housing component 61b) that can be divided into the right and left parts at the axis of the holding tube 21. The left side housing component 61a and the right side housing component 61b are detachably coupled with each other.

At a distal end surface side of the holding tube 21, a knob holding portion 62 configured to hold the rotation operation knob 23 is provided. The rotation operation knob 23 is rotatably coupled with the knob holding portion 62 in the direction about the axis of the outer sheath 18.

A movable handle insertion hole 63 in a long hole shape into which the movable handle 22 is inserted is formed on an upper side portion of a proximal end side surface of the fixed handle 20. As shown in FIG. 5, the movable handle 22 includes an upper arm 22a and a lower arm 22b. The upper arm 22a is extended in a direction perpendicular to the axial direction of the ultrasonic probe 11. The lower arm 22b is bent obliquely downward at a lower end portion of the upper arm 22a in a proximal end direction. A finger insertion ring portion 22c, into which, e.g., a thumb of a user is inserted, is formed at a lower end portion of the lower arm 22b. An elastic ring-shaped finger contact member 22a with a rubber lining is attached to the finger insertion ring portion 22c.

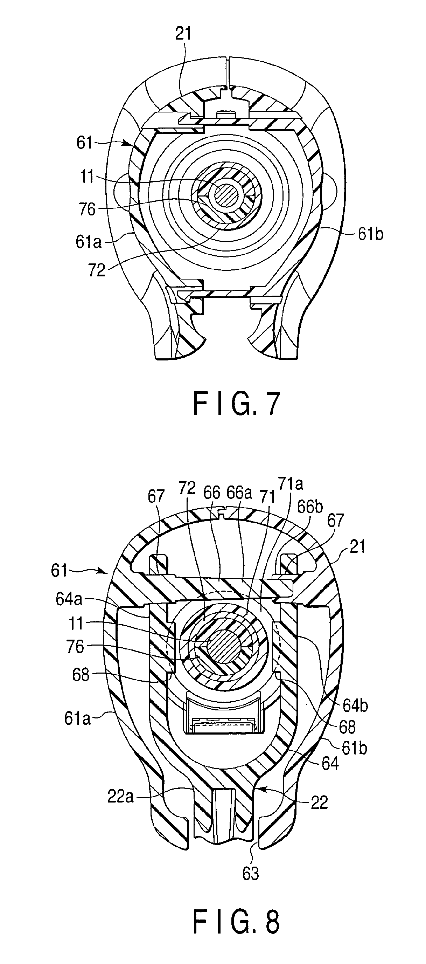

The upper arm 22a is inserted into the operation portion housing 61 through the movable handle insertion hole 63 of the fixed handle 20. As shown in FIG. 8, a U-shaped coupling portion 64 having a substantially U-shape is provided at an upper end portion of the upper arm 22a. The U-shaped coupling portion 64 includes two arms 64a, 64b. The movable handle 22 is attached to a slider member 71 in a state that the slider member 71 of a manipulating force transmission mechanism 70, explained later, is inserted between the two arms 64a, 64b.

A pin insertion hole 67 for a support pin 66 is formed in an upper end portion of each of the arms 64a, 64b. Further, at a lower side part to the pin insertion hole 67 of each of the arms 64a, 64b, an action pin 68 is formed to protrude to the inner side.

The support pin 66 is integrally formed with the operation portion housing 61 in the operation portion housing 61. In this case, a first shaft portion 66a constituting the support pin 66 is formed on the left side housing component 61a. A second shaft portion 66b configured to receive a shaft, into which an end of the first shaft 66a is inserted, is formed in the right side housing component 61b. When the left side housing component 61a and the right side housing component 61b are assembled, the end of the first shaft section 66a is inserted into the second shaft section 66b configured to receive the shaft, so that the first shaft section 66a and the second shaft section 66b are engaged with each other. As a result, the left side housing component 61a and the right side housing component 61b are assembled.

The support pin 66 is inserted into the pin insertion hole 67 of the upper end portion of each of the arms 64a, 64b. Thus, the upper end portion of the movable handle 22 is axially supported by the holding tube 21 via the support pin 66 in a pivotable manner. When the movable handle 22 pivots via the support pin 66, the movable handle 22 is operated to be opened/closed with respect to the fixed handle 20.

As shown in FIG. 5, an operation force transmission mechanism 70 is provided in the holding tube 21 to transmit an operation force applied by the movable handle 22 to the drive pipe 19. The operation force transmission mechanism 70 includes a slider receiving member 72 in a cylindrical shape mainly made of metal and a slider member 71. The slider receiving member 72 is arranged coaxially with the central axis of the holding tube 21, and extended in the same direction as an insertion direction of the ultrasonic probe 11.

A stopper 73 and a spring receiver 74 are provided on an outer peripheral surface of the slider receiving member 72. The stopper 73 is fixed to the outer peripheral surface of a proximal end portion of the slider receiving member 72. The spring receiver 74 is provided on the outer peripheral surface at a distal end side part of the slider receiving member 72 in a protruding manner. The slider member 71 and a coil spring 75 are arranged in the axial direction between the stopper 73 and the spring receiver 74. The stopper 73 is configured to restrict movement of the position the slider member 71 to the proximal end side. A distal end portion of the coil spring 75 is in contact with the spring receiver 74. The coil spring 75 is installed between the spring receiver 74 and the slider member 71 with a constant amount of equipment load.

A ring-shaped engaging groove 71a is formed in a circumferential direction on an outer peripheral surface of the slider member 71. As shown in FIGS. 6 and 8, the action pin 68 of the movable handle 22 is inserted into the engaging groove 71a and engaged therewith. When a user grasps the movable handle 22 to close the movable handle 22 with respect to the fixed handle 20, the action pin 68 pivots about the support pin 66 in accordance with the pivoting movement of the movable handle 22. Concomitantly with the movement of the action pin 68, the slider member 71 moves to a distal direction along the axial direction of the ultrasonic probe 11. At this occasion, the slider receiving member 72 coupled with the slider member 71 via the coil spring 75 also reciprocally moves together with the slider member 71.

A inside of the slider receiving member 72, a tube-shaped probe folder 76 formed of an insulating material is arranged to cover the ultrasonic probe 11. The probe folder 76 is fixed to an inner peripheral surface of the slider receiving member 72. An inner peripheral surface of the probe folder 76 and the outer peripheral surface of the ultrasonic probe 11 are fixed via a rotation stop section, not shown. For example, the rotation stop section includes a key groove formed on one of the inner peripheral surface of the probe folder 76 and the outer peripheral surface of the ultrasonic probe 11, and a key formed on the other of the inner peripheral surface of the probe folder 76 and the outer peripheral surface of the ultrasonic probe 11. In this configuration, the probe folder 76 can move in the axial direction with respect to the ultrasonic probe 11, and the probe folder 76 is restricted such that the probe folder 76 cannot relatively rotate in the direction about the axis with respect to the ultrasonic probe 11.

A proximal end portion of the drive pipe 19 is fixed to a distal end portion of the probe folder 76. Therefore, the operation force of the movable handle 22 is transmitted to the drive pipe 19 via the slider member 71, the coil spring 75, the slider receiving member 72, and the probe folder 76, and the drive pipe 19 moves in the axial direction. When the drive pipe 19 moves, the jaw 17 pivots via the support pin.

As described above, when the drive pipe 19 reciprocally moves in the axial direction, the drive force of the drive pipe 19 is transmitted to the jaw 17 via the coupling pin. Thereby, the jaw 17 is driven to pivot about the support pin. At this occasion, when the drive pipe 19 is operated and pulled to the proximal end side, the jaw 17 is driven to move about the support pin in a direction away from the probe distal end 11a (open position). On the contrary, when the drive pipe 19 is operated and pushed out forward, the jaw 17 is driven to move about the support pin in a direction toward the probe distal end 11a (closed position). When the jaw 17 is operated and pivoted to the closed position, a living tissue is sandwiched between the jaw 17 and the probe distal end 11a of the ultrasonic probe 11.

In the hand piece 1, the treatment section 1A is constituted by the jaw 17, and the probe distal end 11a of the ultrasonic probe 11. In the treatment section 1A, a plurality of, for example, in present embodiment, two treatment functions (a first treatment function and a second treatment function) can be selected. For example, the first treatment function is set as a function of simultaneously outputting an ultrasonic treatment output and a high-frequency treatment output. The second treatment function is set as a function of outputting only the high-frequency treatment output alone.

It should be noted that the first treatment function and the second treatment function of the treatment section 1A are not limited to the above configurations. For example, a configuration may be employed in which the first treatment function is set as a function of outputting the ultrasonic treatment output with a maximum output state, and the second treatment function is set as a function of outputting the ultrasonic treatment output with an output state set in advance which is lower than the maximum output state.

A knob receiving member 77 in a cylindrical shape is arranged on an inner peripheral surface of the rotation operation knob 23. The inner peripheral surface of the rotation operation knob 23 and an outer peripheral surface of the knob receiving member 77 are fixed via a rotation stop section, not shown. The rotation stop section includes a key groove formed on one of the inner peripheral surface of the rotation operation knob 23 and the outer peripheral surface of knob receiving member 77, and a key formed on the other of the inner peripheral surface of the rotation operation knob 23 and the outer peripheral surface of knob receiving member 77. In this configuration, the rotation operation knob 23 and the knob receiving member 77 are restricted such that the rotation operation knob 23 and the knob receiving member 77 cannot relatively rotate in the direction about the axis.

A proximal end portion of the knob receiving member 77 is coupled with the knob holding portion 62 provided at the distal end portion of the holding tube 21 in such a manner that the knob receiving member 77 can rotate about the direction about the axis of the ultrasonic probe 11. Thus, the rotation operation knob 23 is coupled with the knob holding portion 62 of the holding tube 21 in such a manner that the rotation operation knob 23 can rotate in the direction about the axis of the ultrasonic probe 11.

The distal end portion of the probe folder 76 is arranged to the inside of the knob receiving member 77. A pin insertion hole 77a extended in a radial direction is made in the knob receiving member 77, and a pin insertion hole 76a extended in the radial direction is made in the probe folder 76. A coupling pin 78 is inserted into the pin insertion hole 77a of the knob receiving member 77 and the pin insertion hole 76a of the probe folder 76. The knob receiving member 77 and the probe folder 76 are fixed by the coupling pin 78, and are restricted in such a state that they cannot relatively rotate in the direction about the axis with respect to each other.

When the rotation operation knob 23 is operated and rotated, the knob receiving member 77 and the rotation operation knob 23 rotate together. At this occasion, a rotation of the knob receiving member 77 is transmitted to the probe folder 76 via the coupling pin 78. Further, a rotation of the probe folder 76 is transmitted to the ultrasonic probe 11 via the rotation stop section, explained above, disposed between the probe folder 76 and the ultrasonic probe 11. Therefore, the rotation of the rotation operation knob 23 is transmitted to the ultrasonic probe 11 via the knob receiving member 77, the coupling pin 78, and the probe folder 76 in this order, so that the ultrasonic probe 11 as well as the rotation operation knob 23 rotates together. In other words, a rotation torque transmission section 79 is constituted by the rotation operation knob 23, the knob receiving member 77, the coupling pin 78, and the probe folder 76, which is configured to transmit a rotation torque of the rotation operation knob 23 in the direction about the axis of the ultrasonic probe 11 to the ultrasonic probe 11.

In addition, when the rotation operation knob 23 is operated and rotated, the outer sheath 18 as well as the rotation operation knob 23 rotates together in the direction of the axis about the outer sheath 18 with respect to the holding tube 21. Thus, when the rotation operation knob 23 is operated and rotated, the ultrasonic probe 11 and the outer sheath 18 of the sheath body 16 simultaneously rotate in the direction about the axis of the outer sheath 18. Accordingly, the probe distal end 11a of the ultrasonic probe 11 and the jaw 17 are operated and rotated simultaneously in the same direction.

The operation portion housing 61 includes a plural-fingers insertion ring portion 80 at a lower end portion of the fixed handle 20. For example, a plurality of fingers of a user other than a thumb are inserted into the plural-fingers insertion ring portion 80. As shown in FIG. 5, the fixed handle 20 according to the present embodiment includes a switch holding section 81 disposed between the plural-fingers insertion ring portion 80 and the holding tube 21.

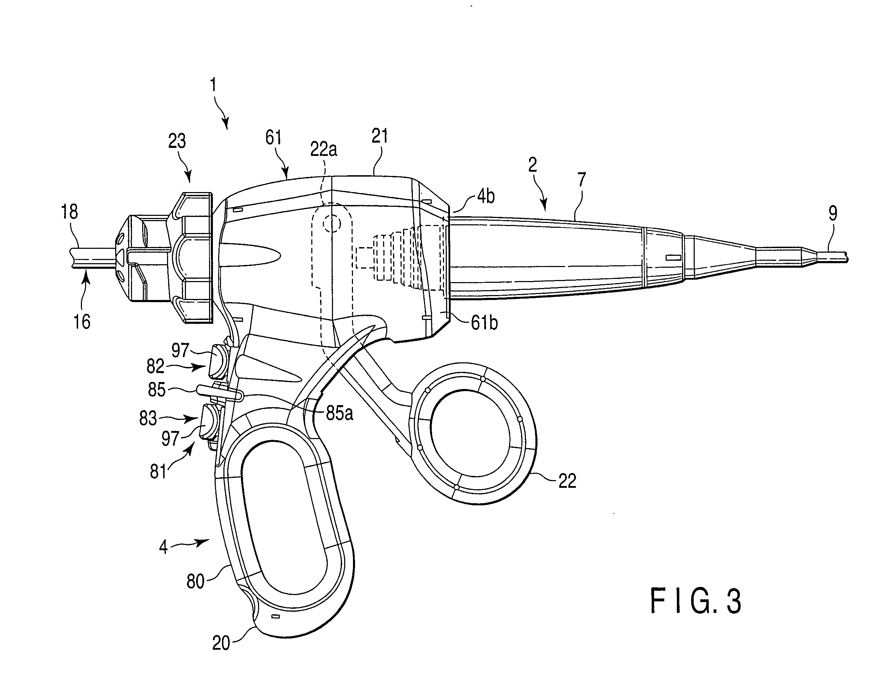

As shown in FIG. 3, the switch holding section 81 includes a switch attachment surface 84 which is provided on a distal end surface of the fixed handle 20, and to which a plurality of, for example, in the present embodiment, two hand switches (a first switch 82 and a second switch 83) are attached. Each of these first switch 82 and second switch 83 is a switch configured to select a treatment function of the treatment section 1A of the hand piece 1.

In the switch holding section 81, the first switch 82 and the second switch 83 are arranged in the vertical direction. Further, a protuberance section 85 is formed between the first switch 82 and the second switch 83 in a protruding manner to the distal end side. The user places a finger on the protuberance section 85.

The first switch 82 is arranged to the upper side of the protuberance section 85. The first switch 82 is set as a switch configured to select the first treatment function which is frequently used among the plural treatment functions.

The second switch 83 is arranged to the lower side of the protuberance section 85. The second switch 83 is set as a switch configured to select the second treatment function which is different from the first treatment function among the plural treatment functions. For example, the first switch 82 is set as a switch button used in coagulation and incision, and the second switch 83 is set as a switch button used in coagulation.

The protuberance section 85 is set in such a manner that the height of the protrusion thereof from the switch attachment surface 84 is more than those of the first switch 82 and the second switch 83. The protuberance section 85 includes an extension portion 85a extended from the switch attachment surface 84 of the fixed handle 20 toward both side surfaces of the fixed handle 20.

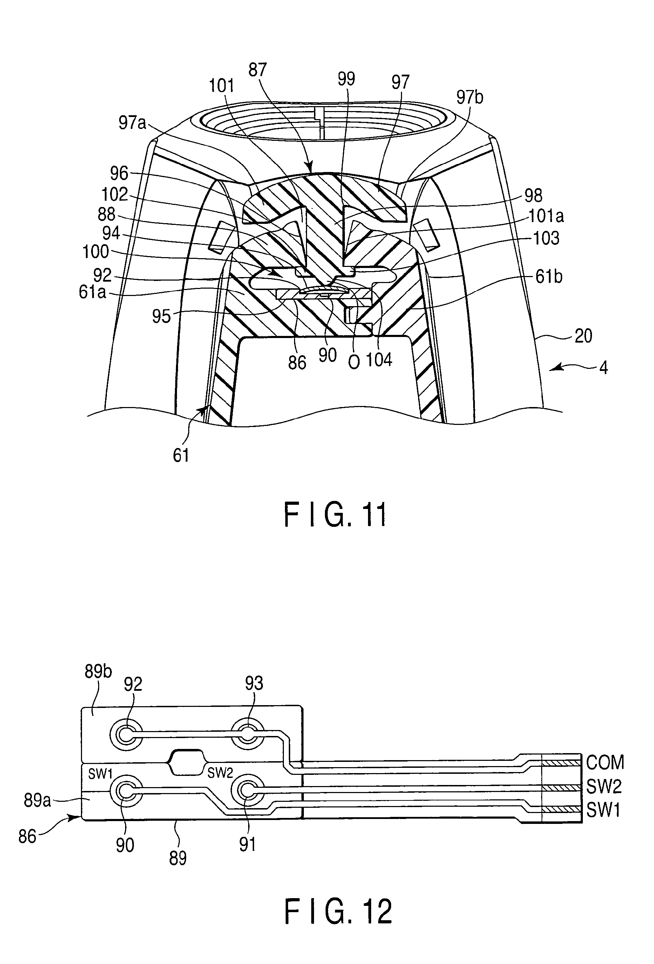

FIG. 11 is a figure illustrating a schematic configuration of the first switch 82 of the hand piece 1. It should be noted that the second switch 83 has the same configuration as the first switch 82. For this reason, only the configuration of the first switch 82 will be hereinafter explained. Description about the configuration of the second switch 83 will not be made.

The first switch 82 includes a switch substrate 86, a switch operation portion 87, and a support portion 88. The switch substrate 86 includes a transmission section, not shown, configured to transmit a signal of driving the apparatus. As shown in FIG. 12, the switch substrate 86 includes a substrate sheet 89 formed of a flexible substrate and the like. The substrate sheet 89 includes a sheet body 89a and a bent portion 89b. The sheet body 89a is provided with a first switch contact point 90 of the first switch 82 and a second switch contact point 91 of the second switch 83. The bent section 89b is provided with a first common contact point 92 of the first switch 82 and a second common contact point 93 of the second switch 83. When the substrate sheet 89 is used, the bent section 89b is bent with respect to the sheet body 89a. Further, the switch substrate 86 includes a dome-shaped metal dome (urging member) 94 made of a metal material provided for each of the first switch 82 and the second switch 83. With the metal dome 94, the switch contact point 90 of the switch substrate 86 is maintained in an open state. When the switch operation portion 87, explained later, is operated, the metal dome 94 is elastically deformed and squashed, whereby the switch contact point 90 is closed. As a result, the first switch contact point 90 of the first switch 82 and the first common contact point 92 are connected, and the transmission section of the first switch 82 of the switch substrate 86 transmits the signal of driving the apparatus.

The operation portion housing 61 is provided with a substrate holding section 95 configured to hold the switch substrate 86 and a switch operation portion holding section 96 configured to hold the switch operation portion 87. The substrate holding section 95 holds the switch substrate 86 at position corresponding to the first switch 82 and the second switch 83 in the operation portion housing 61.

The switch operation portion 87 includes a pressure-receiving portion 97 configured to be pressed by the user. The pressure-receiving portion 97 is supported by the support portion 88 in such a manner that the pressure-receiving portion 97 can move about an inclination movement center O in the operation portion housing 61 to incline with respect to the operation portion housing 61. During pressing operation in which the pressure-receiving portion 97 is pressed, the support portion 88 supports the pressure-receiving portion 97 in such a state that the pressure-receiving portion 97 can be pressed in a direction where the inclination movement center O of the pressure-receiving portion 97 is arranged against a force applied by the metal dome 94. On the other hand, during non-pressing operation of the pressure-receiving portion 97, the support portion 88 holds the switch operation portion 87 at a position where the switch contact point 90 is held in an open state.

The switch operation unit 87 includes a shaft section 98. At one end portion of the shaft section 98 (end portion on a side of the pressure-receiving unit 97), a first coupling section 99 coupling with the pressure-receiving portion 97 is provided. At the other end portion of the shaft section 98 (end portion on a side of the switch contact point 90), a second coupling section 100 coupling with the operation portion housing 61 is provided.

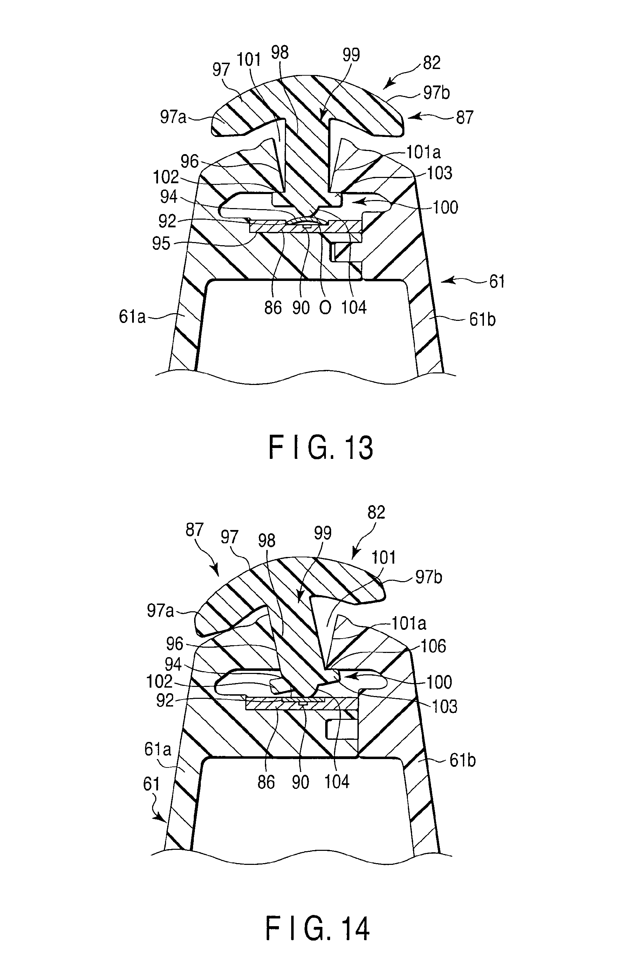

The support portion 88 includes a clearance formation section 101 configured to form a clearance between the support portion 88 and a portion of the shaft section 98 of the switch operation portion 87 on a side of the first coupling section 99. With the clearance formed by the clearance formation section 101, the pressure-receiving portion 97 can move about the inclination movement center O of the pressure-receiving portion 97 in inclination movement directions. The clearance formation section 101 includes an inclination surface 101a whose size of cross sectional area of the clearance gradually increases from a switch contact point 90 side to a pressure-receiving portion 97 side. FIG. 13 is a figure illustrating the pressure-receiving portion 97 of the first switch 82 maintained in the non-pressed state when the pressure-receiving portion 97 and the shaft section 98 are maintained in a neutral position without inclination. In this case, the pressure-receiving portion 97 and the shaft section 98 are restricted such that the pressure-receiving portion 97 and the shaft section 98 are inclined from the neutral position to inclination states in two directions, i.e., a first inclination movement direction in which the pressure-receiving portion 97 and the shaft section 98 are inclined in the left direction in FIG. 13 and a second inclination movement direction in which the pressure-receiving portion 97 and the shaft section 98 are inclined in the right direction in FIG. 13. As shown in FIG. 5, inclination in a direction perpendicular to the inclination movement directions of the pressure-receiving portion 97 and the shaft section 98 is restricted by wall surfaces of the operation portion housing 61 configured to come into contact with the shaft section 98. For this reason, the inclination movement directions of the pressure-receiving portion 97 are restricted to only two directions, i.e., a first inclination movement direction in which the pressure-receiving portion 97 inclines from the neutral position to the left side in FIG. 13 and a second inclination movement direction in which the pressure-receiving portion 97 inclines from the neutral position to the right side in FIG. 13.

The pressure-receiving portion 97 of the first switch 82 is a push button formed in a long arc shape, in which the pressing surface is formed along two directions, i.e., the first inclination movement direction and the second inclination movement direction. A first coupling section 99, serving as a coupling section with the shaft section 98, is provided at a central position of the arc-shaped pressing surface of the pressure-receiving portion 97. The pressure-receiving portion 97 includes a left side extension section (first inclination movement direction extension section) 97a extended from the first coupling section 99 to the left side in FIG. 13 (first inclination movement direction) and a right side extension section (second inclination movement direction extension section) 97b extended from the first coupling section 99 to the right side in FIG. 13 (second inclination movement direction).

The second coupling section 100 includes a first protrusion 102 protruding in the first inclination movement direction, a second protrusion 103 protruding in the second inclination movement direction, and a switch contact section 104 configured to be in contact with the metal dome 94 of the switch substrate 86.

When the user straightly presses the pressure-receiving portion 97 of the first switch 82 from the neutral position of FIG. 13, the switch contact section 104 presses the metal dome 94 of the switch substrate 86. Accordingly, the metal dome 94 of the first switch 82 is elastically deformed and squashed. As a result, the first switch contact point 90 of the first switch 82 and the first common contact point 92 are brought into contact, whereby the switch contact point 90 of the first switch 82 is closed.

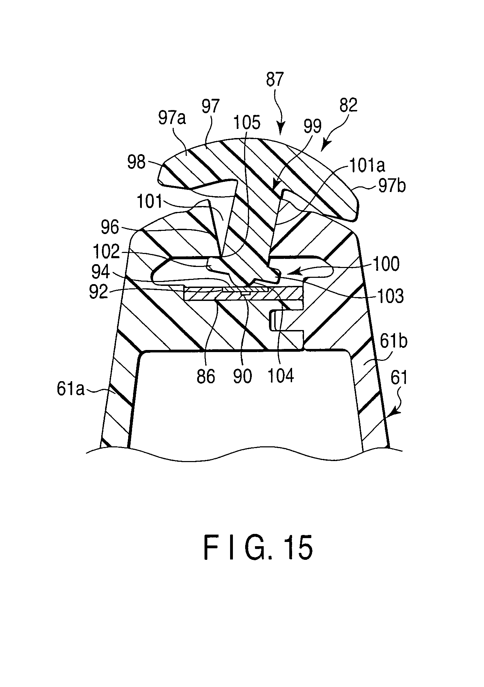

As shown in FIG. 15, when the right side extension section (second inclination movement direction extension section) 97b of the pressure-receiving portion 97 is pressed, the pressure-receiving portion 97 of the first switch 82 is inclined and moved in the second inclination movement direction. At this occasion, a first inclination movement support point 105 is formed at a contact section between the first protrusion 102 and the inner surface of the operation portion housing 61. When the shaft section 98 is inclined and moved about the first inclination movement support point 105, the switch contact section 104 presses the metal dome 94 of the switch substrate 86. Accordingly, the metal dome 94 of the first switch 82 is elastically deformed and squashed. As a result, the first switch contact point 90 of the first switch 82 and the first common contact point 92 are brought into contact, whereby the switch contact point 90 of the first switch 82 is closed.

On the other hand, as shown in FIG. 14, when the left side extension section (first inclination movement direction extension section) 97a of the pressure-receiving portion 97 is pressed, the pressure-receiving portion 97 of the first switch 82 is inclined and moved in the first inclination movement direction. At this occasion, a second inclination movement support point 106 is formed at a contact section between the second protrusion 103 and the inner surface of the operation portion housing 61. When the shaft section 98 is inclined and moved about the second inclination movement support point 106, the switch contact section 104 presses the metal dome 94 of the switch substrate 86. Accordingly, the metal dome 94 of the first switch 82 is elastically deformed and squashed. As a result, the first switch contact point 90 of the first switch 82 and the first common contact point 92 are brought into contact, whereby the switch contact point 90 of the first switch 82 is closed.

It should be noted that the second switch 83 is operated in the same manner as the first switch 82. Accordingly, the metal dome 94 of the second switch 83 is elastically deformed and squashed. As a result, the second switch contact point 91 of the second switch 82 and the second common contact point 93 are connected, whereby the switch contact point 91 of the second switch 83 is closed. Accordingly, the transmission section of the second switch 83 of the switch substrate 86 transmits the signal of driving the apparatus.

It should be noted that a rubber cover which covers the entire outer peripheral surface of the pressure-receiving portion 97 of the first switch 82 and a rubber cover which covers the entire outer peripheral surface of the pressure-receiving portion 97 of the second switch 83 may be provided on the operation portion housing 61. This makes a waterproof sealing structure in the first switch 82 and the second switch 83.

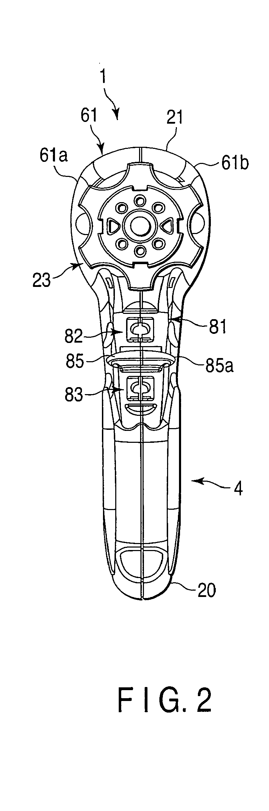

Subsequently, the function of the present embodiment will be explained. As shown in FIG. 2, in the hand piece 1 of the surgical operating apparatus according to the present embodiment, two units, i.e., the vibrator unit 2 and the handle unit 4, are detachably coupled. When the hand piece 1 is used, the vibrator unit 2 and the handle unit 4 are coupled to each other. As a result of this, in the connected body of the vibrator unit 2 and the ultrasonic probe 11, a first high-frequency electric pathway through which a high-frequency current is configured to be transmitted is formed.

On the other hand, in the hand piece 1, a second high-frequency electric pathway through which a high-frequency current is configured to be transmitted to the electrode member of the jaw 17 is formed. When the high-frequency current is transmitted to the electrode member of the jaw 17, the second electrode section of the bipolar electrode is formed. As described above, the hand piece 1 is assembled.

When the hand piece 1 is used, the movable handle 22 is operated to be opened or closed with respect to the fixed handle 20. The drive pipe 19 is moved in the axial direction in accordance with the operation of the movable handle 22, and the jaw 17 is driven to be opened or closed with respect to the probe distal end 11a of the ultrasonic probe 11 in accordance with the advancing/retreating operation of the drive pipe 19 in the axial direction. In this case, when the movable handle 22 is operated to be closed with respect to the fixed handle 20, the drive pipe 19 is pushed to the distal end side in accordance with the operation of the movable handle 22. The jaw 17 is driven in a direction in which the jaw 17 is made closer to the probe distal end 11a of the ultrasonic probe 11 (closed position) in accordance with the pushing operation of the drive pipe 19. When the jaw 17 is operated and pivoted to the closed position, a living tissue is sandwiched between the jaw 17 and the probe distal end 11a of the ultrasonic probe 11.

At this state, any one of the first switch 82 and the second switch 83 of the fixed handle 20 is selectively pushed. When the first switch 82 is pushed, a drive current is passed through the ultrasonic vibrator 6 and the ultrasonic vibrator 6 is driven, and simultaneously the high-frequency current is turned on. At this time, the ultrasonic vibration from the ultrasonic vibrator 6 is transmitted to the probe distal end 11a through a vibration transmission member 11. As a result of this, it is possible to perform a treatment such as incision, resection, and the like of the living tissue by utilizing the ultrasonic waves together with the application of the high-frequency waves. It should be noted that it is also possible to perform a coagulation treatment of the living tissue by utilizing the ultrasonic waves.

When the first switch 82 is pressed, and the pressure-receiving portion 97 of the first switch 82 is straightly pressed from the neutral position at which the pressure-receiving portion 97 is not inclined as shown in FIG. 13, the switch contact section 104 presses the metal dome 94 of the switch substrate 86. Accordingly, the metal dome 94 of the first switch 82 is elastically deformed and squashed. As a result, the first switch contact point 90 of the first switch 82 and the first common contact point 92 are brought into contact, whereby the switch contact point 90 of the first switch 82 is closed. Accordingly, the transmission section of the first switch 82 of the switch substrate 86 transmits the signal of driving the apparatus.

As shown in FIG. 15, when the right side extension section (second inclination movement direction extension section) 97b of the pressure-receiving portion 97 is pressed, the pressure-receiving portion 97 of the first switch 82 is inclined and moved in the second inclination movement direction. At this occasion, a first inclination movement support point 105 is formed at a contact section between the first protrusion 102 and the inner surface of the operation portion housing 61. When the shaft section 98 is inclined and moved about the first inclination movement support point 105, the switch contact section 104 presses the metal dome 94 of the switch substrate 86. Accordingly, the metal dome 94 of the first switch 82 is elastically deformed and squashed. As a result, the first switch contact point 90 of the first switch 82 and the first common contact point 92 are brought into contact, whereby the switch contact point 90 of the first switch 82 is closed. Accordingly, the transmission section of the first switch 82 of the switch substrate 86 transmits the signal of driving the apparatus

On the other hand, as shown in FIG. 14, when the left side extension section (first inclination movement direction extension section) 97a of the pressure-receiving portion 97 is pressed, the pressure-receiving portion 97 of the first switch 82 is inclined and moved in the first inclination movement direction. At this occasion, a second inclination movement support point 106 is formed at a contact section between the second protrusion 103 and the inner surface of the operation portion housing 61. When the shaft section 98 is inclined and moved about the second inclination movement support point 106, the switch contact section 104 presses the metal dome 94 of the switch substrate 86. Accordingly, the metal dome 94 of the first switch 82 is elastically deformed and squashed. As a result, the first switch contact point 90 of the first switch 82 and the first common contact point 92 are brought into contact, whereby the switch contact point 90 of the first switch 82 is closed. Accordingly, the transmission section of the first switch 82 of the switch substrate 86 transmits the signal of driving the apparatus.

Therefore, in the present embodiment, the user can perform the switch operation which is adapted to cause the transmission section of the first switch 82 of the switch substrate 86 to transmit the signal of driving the apparatus no matter at which position of the arc-shaped pressing surface of the pressure-receiving portion 97 of the first switch 82 the user presses the pressure-receiving portion 97.

When the user cancels pressing of the pressure-receiving portion 97 of the first switch 82, the metal dome 94 of the first switch 82 elastically recovers back to its original dome shape, so that the connection between the first switch contact point 90 of the first switch 82 and the first common contact point 92 is disconnected, whereby the switch contact point 90 of the first switch 82 is opened.

When the second switch 83 is pressed, the first high-frequency electric path through which the high-frequency current flows to the probe distal end 11a of the ultrasonic probe 11 and the second high-frequency electric path through which the high-frequency current flows to the jaw body of the jaw 17 are conducted. Accordingly, two bipolar electrodes used in high-frequency treatment are constituted by the probe distal end 11a of the ultrasonic probe 11 and the jaw body. By causing a high-frequency current to flow between the two bipolar electrodes that are the probe distal end 11a of the ultrasonic probe 11 and the jaw 17, a high-frequency treatment by the bipolar electrodes can be applied to the living tissue between the jaw 17 and the probe distal end 11a of the ultrasonic probe 11.

It should be noted that the second switch 83 is operated in the same manner as the first switch 82. Accordingly, the metal dome 94 of the second switch 83 is elastically deformed and squashed. As a result, the second switch contact point 91 of the second switch 82 and the second common contact point 93 are connected, whereby the switch contact point 91 of the second switch 83 is closed. Accordingly, the transmission section of the second switch 83 of the switch substrate 86 transmits the signal of driving the apparatus.

When the movable handle 22 is operated to be opened with respect to the fixed handle 20, the drive pipe 19 is pulled to the proximal end side in accordance with the operation of opening the movable handle 22. When the drive pipe 19 is pulled, the jaw 17 is driven to move in a direction away from the probe distal end 11a of the ultrasonic probe 11 (open position).

Therefore, the hand piece 1 having the above configuration achieves the following effects. That is, the hand piece 1 of the present embodiment is provided with the support portion 88 configured to support the pressure-receiving portion 97 of the first switch 82 in such a manner that the pressure-receiving portion 97 of the first switch 82 can incline about the inclination movement center O in the operation portion housing 61. Accordingly, when the user presses a part away from the central position of the arc-shaped pressing surface of the pressure-receiving portion 97 of the first switch 82, the pressure-receiving portion 97 can be inclined and moved about the inclination movement center O in the operation portion housing 61. When the pressure-receiving portion 97 is inclined and moved with respect to the operation portion housing 61, the pressing surface of the pressure-receiving portion 97 of the first switch 82 is moved, so that the operator can press the pressing surface of the pressure-receiving portion 97 while the operator relaxes the fingers to easily press the pressure-receiving portion 97. In other words, the pressure-receiving portion 97 can be inclined and moved within the movable range of the finger of the user. As a result, unlike a conventional medical apparatus, after the operator moves the finger to the position where the switch is provided so as to press the switch, the operator does not have to straightly press an activation rod of the switch in an axial direction of the switch while the operator maintains the posture of the finger without changing the posture of the finger so as to easily press the switch. This improves the ease of operation and the feeling of pressing operation when the operator operates the first switch 82.

In addition, in the present embodiment, the pressure-receiving portion 97 of each of the first switch 82 and the second switch 83 is a push button whose pressing surface is formed in a long arc shape along two directions, i.e., the first direction and the second direction. In this configuration, no matter at which position of the arc-shaped pressing surface the pressure-receiving portion 97 of each of the first switch 82 and the second switch 83 is pressed, the user can perform the switch operation of causing the transmission section of the first switch 82 or the second switch 83 of the switch substrate 86 to transmit the signal of driving the apparatus. For this reason, even when the user pushes a position of the pressure-receiving portion 97 away from the central position of the pressure-receiving portion 97 during operation of the first switch 82 or the second switch 83, the switch can be operated. Therefore, the user's ease of operation can be improved, and the switch can be operated stably.

In addition, in the present embodiment, when the pressure-receiving portion 97 of the first switch 82 or the second switch 83 is pressed, the metal dome 94 of the first switch 82 or the second switch 83 is elastically deformed and squashed, whereby a click sound is generated. Therefore, the operator can feel the click, which reliably allows the operator to confirm the switch operation of the first switch 82 or the second switch 83.

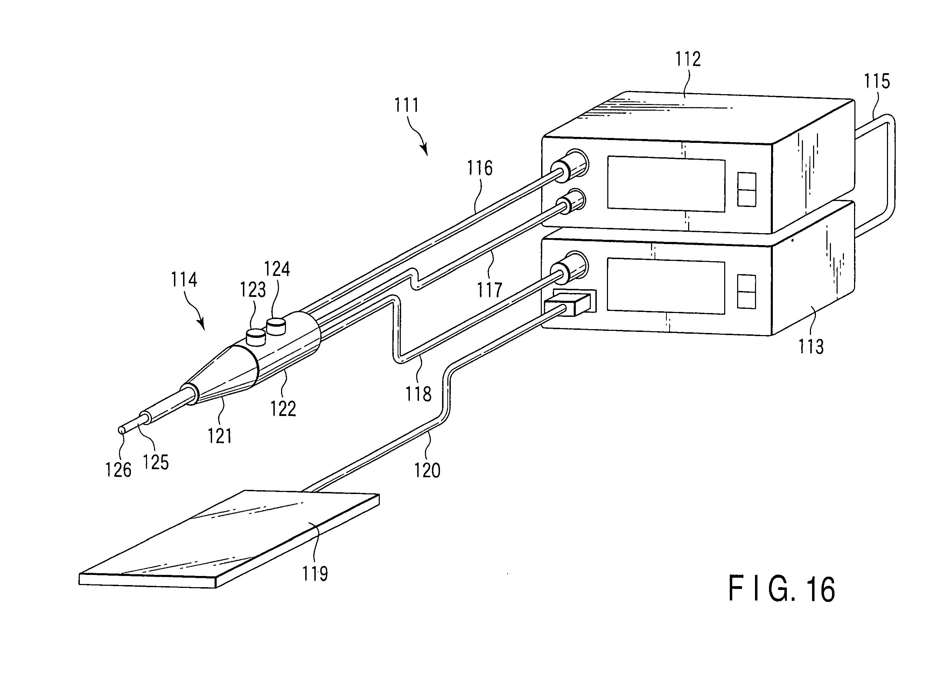

FIGS. 16 to 18 are figures illustrating the second embodiment of the present invention. In the present embodiment, a surgical apparatus 111 having a configuration different from that of the surgical operating apparatus according to the first embodiment (see FIGS. 1 to 15) is used as an apparatus.

The surgical apparatus 111 mainly includes an ultrasonic drive device 112, a high-frequency drive device 113, and a hand piece 114. The ultrasonic drive device 112 and the high frequency drive device 113 are connected to each other by way of a communication cable 115.

The hand piece 114 is connected to the ultrasonic drive device 112 by way of an output connection cable 116 and an SW connection cable 117 via a connector. The hand piece 114 is connected to the high frequency drive device 113 by way of an output connection cable 118 via a connector. A counter electrode plate 119 is connected to the high frequency drive device 113 by way of a connection cable 120 via a connector.

The hand piece 114 includes a sheath body 121 provided at a distal end side part and a substantially cylindrical-shaped operation portion housing 122 provided at a proximal end side part. The operation portion housing 122 and the sheath body 121 are fitted into each other. The operation portion housing 122 includes two housing components (a left side housing component 122a and a right side housing component 122b) that can be divided into the right-and-left parts. The left side housing component 122a and the right side housing component 122b are detachably coupled with each other.

An ultrasonic vibrator (e.g., bolt-clamped Langevin type vibrator), not shown, is provided in the operation portion housing 122 configured to generate ultrasonic vibration. Electric power used in driving an ultrasonic wave is supplied from the ultrasonic drive device 112 to the ultrasonic vibrator via the output connection cable 116.

A probe 125 configured to transmit ultrasonic vibration is screwed to a distal end side of the ultrasonic vibrator. A treatment section 126 is arranged at a distal end portion of the probe 125 in order to treat living tissue.

Further, the operation portion housing 122 is provided with a first switch 123 and a second switch 124 as a hand switch. The first switch 123 and the second switch 124 are mounted on a switch substrate 131 which is an electric circuit substrate accommodated in the operation portion housing 122. A lead in the SW connection cable 117 is connected to a connection terminal of the switch substrate 131 typically by soldering. The SW connection cable 117 is extended externally from a proximal end side of the operation portion housing 122 and is connected to the ultrasonic drive device 112 via a connector. With this arrangement, the electric signals of the first switch 123 and the second switch 124 can be transmitted to the ultrasonic drive device 112 by way of the SW connection cable 117.

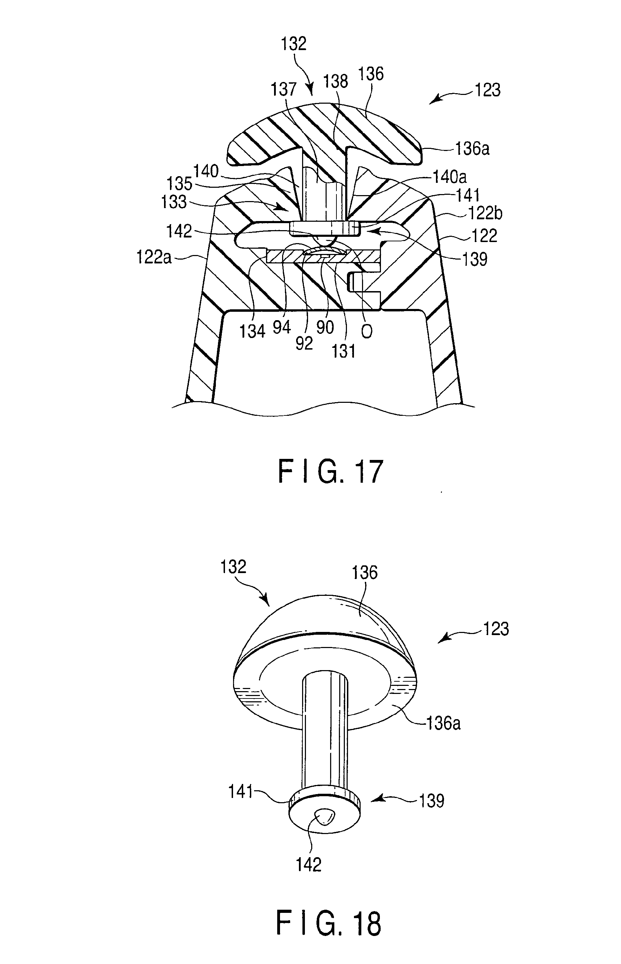

FIG. 17 is a figure illustrating a schematic configuration of the first switch 123 of the hand piece 114. It should be noted that the second switch 124 has the same configuration as the first switch 123. Therefore, for this reason, only the configuration of the first switch 123 will be hereinafter explained. Description about the configuration of the second switch 124 will not be made.

The first switch 124 includes a switch substrate 131, a switch operation portion 132, and a support portion 133. Since the switch substrate 131 has the same configuration as the switch substrate 86 according to the first embodiment, the same parts as those of the switch substrate 86 of the first embodiment are denoted with the same reference numerals and description thereof will not be made.

The operation portion housing 122 is provided with a substrate holding section 134 configured to hold the switch substrate 131 and a switch operation portion holding section 135 configured to hold the switch operation portion 132. The substrate holding section 134 holds the switch substrate 131 at position corresponding to the first switch 123 and the second switch 124 in the operation portion housing 122.

The switch operation portion 132 includes a pressure-receiving portion 136 configured to be pressed by the user. The pressure-receiving portion 132 is supported by the support portion 133 in such a manner that the pressure-receiving portion 132 can move about an inclination movement center 0 in the operation portion housing 122 to incline with respect to the operation portion housing 122. During pressing operation in which the pressure-receiving portion 136 is pressed, the support portion 133 supports the pressure-receiving portion 136 in such a state that the pressure-receiving portion 136 can be pressed in a direction where the inclination movement center 0 of the pressure-receiving portion 136 is arranged against the force applied by the metal dome 94. On the other hand, during non-pressing operation of the pressure-receiving portion 136, the support portion 133 holds the switch operation portion 132 at a position where the switch contact point 90 is held in an open state. The switch operation portion 132 includes a shaft section 137. At one end portion of the shaft section 137 (end portion on the side of the pressure-receiving portion 136), a first coupling section 138 coupling with the pressure-receiving portion 136 is provided. At the other end portion of the shaft section 137 (end portion on the side of the switch contact point 90), a second coupling section 139 coupling with the operation portion housing 122 is provided.

The support portion 133 includes a clearance formation section 140 configured to form a clearance between the support portion 133 and a first coupling section 138 side part of the shaft section 137 of the switch operation portion 132. With the clearance formed by the clearance formation section 140, the pressure-receiving portion 136 can move about the inclination movement center O of the pressure-receiving portion 136 in the inclination movement directions. The clearance formation section 140 includes a cone-shaped inclination surface 140a whose size of cross sectional area of the clearance gradually increases from a switch contact point 90 side to a pressure-receiving unit 136 side. FIG. 17 is a figure illustrating the pressure-receiving portion 136 of the first switch 123 maintained in the non-pressed state when the pressure-receiving portion 136 and the shaft section 137 are maintained in a neutral position without inclination. In this case, the shaft section 137 of the pressure-receiving portion 136 is maintained in a state in which the shaft section 137 can move and incline in any given directions from the neutral position.

The pressure-receiving portion 136 of the first switch 123 is a push button whose pressing surface is substantially formed in a dome shape. At the dome-shaped central position of the pressure-receiving portion 136, the first coupling section 138 with the shaft section 137 is provided. The pressure-receiving portion 136 includes an outer-peripheral-directions extension section 136a extended in an outer peripheral directions from the first coupling section 138.

The second coupling section 139 includes a flange-shaped protrusion 141 arranged to protrude from the shaft section 137 in the outer peripheral directions, and also includes a switch contact section 142 configured to be in contact with the metal dome 94 of the switch substrate 131.

When the user straightly presses the pressure-receiving portion 136 of the first switch 123 at the neutral position of FIG. 17, the switch contact section 142 presses the metal dome 94 of the switch substrate 131. Accordingly, the metal dome 94 of the first switch 123 is elastically deformed and squashed. As a result, the first switch contact point 90 of the first switch 123 and the first common contact point 92 are brought into contact, whereby the switch contact point 90 of the first switch 123 is closed. Accordingly, the transmission section of the first switch 123 of the switch substrate 131 transmits the signal of driving the apparatus.

When a user presses an end edge position of the outer-peripheral-directions extension section 136a that is displaced in any one of a given directions from the central position of the pressure-receiving portion 136, the pressure-receiving portion 136 of the first switch 123 is inclined and moved in a direction in which the pressed position resides with respect to the central position of the pressure-receiving portion 136 (pressed position direction). At this occasion, the shaft section 137 inclines and moves in one of the inclination movement directions of the pressure-receiving portion 136, and at an end edge portion on the side opposite to said one of the inclination movement directions of the pressure-receiving portion 136 with respect to the inclination movement center O of the pressure-receiving portion 136, an inclination movement support point is formed at a contact section between the protrusion 141 and the inner surface of the operation portion housing 122. When the shaft section 137 is inclined and moved about the inclination movement support point, the switch contact section 142 presses the metal dome 94 of the switch substrate 131. Accordingly, the metal dome 94 of the first switch 123 is elastically deformed and squashed. As a result, the first switch contact point 90 of the first switch 123 and the first common contact point 92 are brought into contact, whereby the switch contact point 90 of the first switch 123 is closed. Accordingly, the transmission section of the first switch 123 of the switch substrate 131 transmits the signal of driving the apparatus.

It should be noted that the second switch 124 is operated in the same manner as the first switch 123. Accordingly, the metal dome 94 of the second switch 124 is elastically deformed and squashed. As a result, the second switch contact point 91 of the second switch 124 and the second common contact point 93 are connected, whereby the switch contact point 91 of the second switch 124 is closed. Accordingly, the transmission section of the second switch 124 of the switch substrate 131 transmits the signal of driving the apparatus.

Therefore, the surgical apparatus 111 having the above configuration achieves the following effects. That is, the hand piece 114 of the surgical apparatus 111 of the present embodiment is provided with the support portion 133 configured to support the pressure-receiving portion 136 of the first switch 123 in such a manner that the pressure-receiving portion 136 of the first switch 123 can incline and move about the inclination movement center O in the operation portion housing 122. Accordingly, when the user presses a position away from the central position of the dome-shaped pressing surface of the pressure-receiving portion 136 of the first switch 123, the pressure-receiving portion 136 can be inclined and moved about the inclination movement center O in the operation portion housing 122. When the pressure-receiving portion 136 is inclined and moved with respect to the operation portion housing 122, the pressing surface of the pressure-receiving portion 136 of the first switch 123 is moved, so that the operator can press the pressing surface of the pressure-receiving portion 136 while the operator relaxes the fingers to easily press the pressure-receiving portion 136. In other words, the pressure-receiving portion 136 can be inclined and moved within the movable range of the finger of the user. This improves the ease of operation and the feeling of pressing operation when the operator operates the first switch 123.

Further, in the present embodiment, the pressure-receiving portion 136 of each of the first switch 123 and the second switch 124 is a push button whose pressing surface is formed in a dome shape. In this configuration, no matter at which position of the dome-shaped pressing surface (push button) the pressure-receiving portion 136 of each of the first switch 123 and the second switch 124 is pressed, the user can perform the switch operation adapted to cause the transmission section of the first switch 123 or the second switch 124 of the switch substrate 131 to transmit the signal of driving the apparatus. For this reason, even when the user pushes a position of the pressure-receiving portion 136 away from the central position of the pressure-receiving portion 136 during operation of the first switch 123 or the second switch 124, the switch can be operated. Therefore, the user's ease of operation can be improved, and the switch can be operated stably.

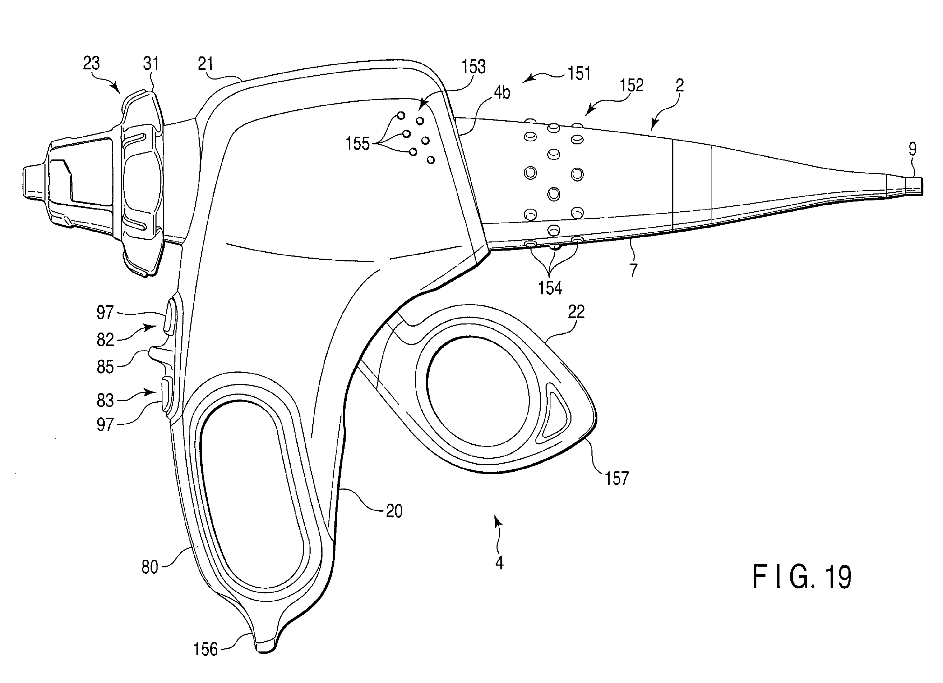

FIG. 19 is a figure illustrating a hand piece 151 of a surgical operating apparatus according to the third embodiment of the present invention. The hand piece 151 of the surgical operating apparatus according to the present embodiment is the same ultrasonic coagulation-incision treatment apparatus as that of the first embodiment (see FIGS. 1 to 15). The configurations of the basic parts are the same as the first embodiment. However, details of the configuration differ from the first embodiment. In FIG. 19, the same portions as those of the first embodiment are denoted with the same reference numerals and description thereof will not be made.

More specifically, in the present embodiment, external display portions 152, 153 having the same kind of design are provided at a coupling portion between a vibrator unit 2 and a handle unit 4. In this case, the first external display portion 152 of the vibrator unit 2 is provided on an outer peripheral surface at a distal end portion of a vibrator cover 7. In the first external display portion 152, a plurality of dot-shaped protrusions 154 are arranged along a circumferential direction.

The second external display portion 153 of the handle unit 4 is arranged at a position nearby a vibrator connection portion 4b of a holding tube 21. The second external display portion 153 is provided on both side surfaces of a proximal end portion of the holding tube 21. In the second external display portion 153, a plurality of dot-shaped protrusions 155 are arranged side by side. The first external display portion 152 of the vibrator unit 2 and the second external display portion 153 of the handle unit 4 have the same design to give the sense of unity. Accordingly, it is easy to confirm that the vibrator unit 2 and the handle unit 4 are of the same model.

In a fixed handle 20, a nail-shaped finger placing portion 156 is provided at a lower end portion of a plural-fingers insertion ring section 80 in a protruding manner. Likewise, at one end portion of a movable handle 22, a protruding portion 157 substantially in a triangular shape is provided in a protruding manner.

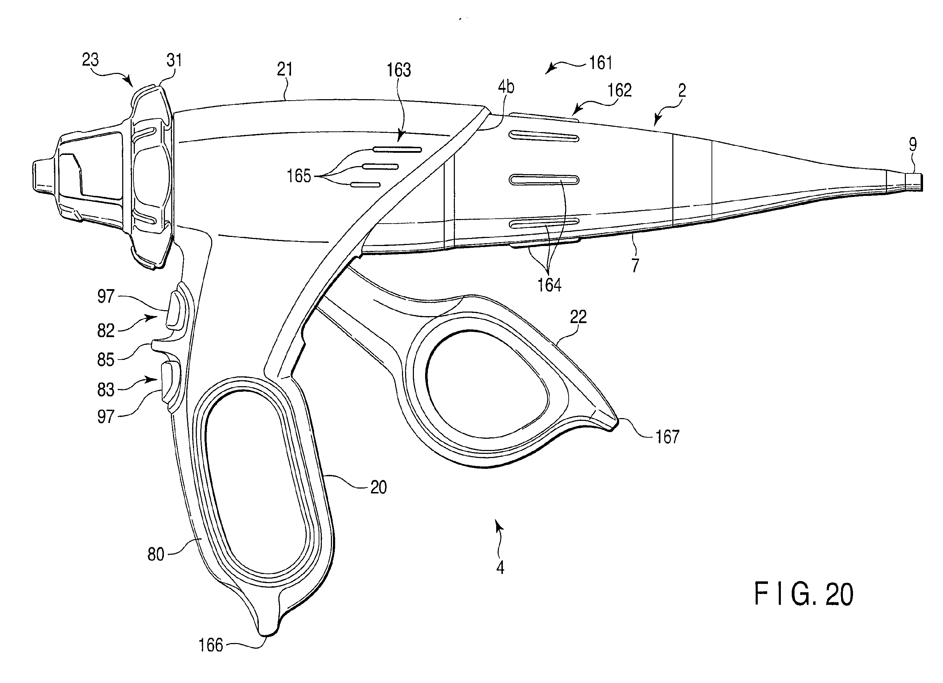

FIG. 20 is a figure illustrating a hand piece 161 of a surgical operating apparatus according to the fourth embodiment of the present invention. The hand piece 161 of the surgical operating apparatus according to the present embodiment is the same ultrasonic coagulation-incision treatment apparatus as that of the first embodiment (see FIGS. 1 to 15). The configurations of the basic parts are the same as the first embodiment. However, details of the configuration differ from the first embodiment. In FIG. 20, the same portions as those of the first embodiment are denoted with the same reference numerals and description thereof will not be made.

More specifically, in the present embodiment, external display portions 162, 163 having the same kind of design are provided at a coupling portion between a vibrator unit 2 and a handle unit 4. The external display portions 162, 163 are formed in different shapes from the external display portions 152, 153 of the hand piece 151 according to the third embodiment (see FIG. 19).

In this case, the first external display portion 162 of the vibrator unit 2 is provided on an outer peripheral surface at a distal end portion of a vibrator cover 7. In the first external display portion 162, a plurality of straight protrusions 164 are arranged along a circumferential direction side by side. Each protrusion 164 is extended substantially in parallel with an axial direction.

The second external display portion 163 of the handle unit 4 is arranged at a position nearby a vibrator connection portion 4b of a holding tube 21. The second external display portion 163 is provided on both side surfaces of a proximal end portion of the holding tube 21. In the second external display portion 163, a plurality of straight protrusions 165 are arranged side by side. The first external display portion 162 of the vibrator unit 2 and the second external display unit 163 of the handle unit 4 have the same design to give the sense of unity. Accordingly, it is easy to confirm that the vibrator unit 2 and the handle unit 4 are of the same model

In a fixed handle 20, a nail-shaped finger placing portion 166 is provided at a lower end portion of a plural-fingers insertion ring section 80 in a protruding manner. Likewise, at one end portion of a movable handle 22, a nail-shaped finger placing portion 167 is provided in a protruding manner.

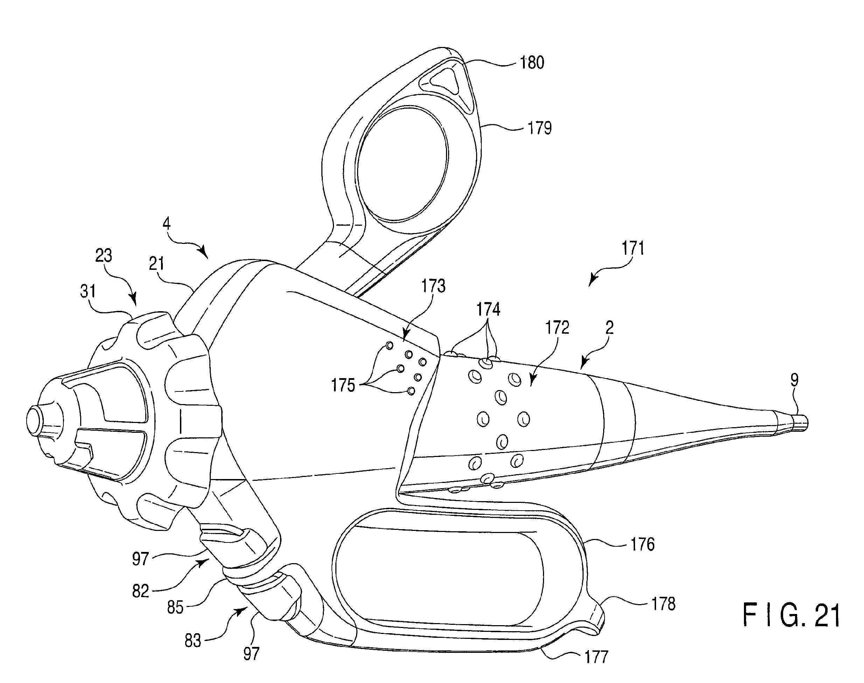

FIG. 21 illustrates a hand piece 171 of a surgical operating apparatus according to the fifth embodiment of the present invention. The present embodiment is a modification of the hand piece 151 according to the third embodiment (see FIG. 19). The hand piece 171 of the surgical operating apparatus according to the present embodiment is the same ultrasonic coagulation-incision treatment apparatus as that of the first embodiment (see FIGS. 1 to 15). The configurations of the basic parts are the same as the first embodiment. However, details of the configuration differ from the first embodiment. In FIG. 21, the same portions as those of the first embodiment are denoted with the same reference numerals and description thereof will not be made.

More specifically, in the present embodiment, external display portions 172, 173 having the same kind of design are provided at a coupling portion between a vibrator unit 2 and a handle unit 4. In this case, the first external display portion 172 of the vibrator unit 2 has substantially the same configuration as the first external display unit 152 of the vibrator unit 2 of the hand piece 151 according to the third embodiment (see FIG. 19). The first external display portion 172 of the vibrator unit 2 is provided on an outer peripheral surface at a distal end portion of a vibrator cover 7. In the first external display portion 172, a plurality of dot-shaped protrusions 174 are arranged along a circumferential direction.

The second external display portion 173 of the handle unit 4 is arranged at a position nearby a vibrator connection portion 4b of a holding tube 21. The second external display portion 173 is provided on both side surfaces of a proximal end portion of the holding tube 21. In the second external display portion 173, a plurality of dot-shaped protrusions 175 are arranged side by side. The first external display portion 172 of the vibrator unit 2 and the second external display unit 173 of the handle unit 4 have the same design to give the sense of unity. Accordingly, it is easy to confirm that the vibrator unit 2 and the handle unit 4 are of the same model. The second external display portion 173 of the handle unit 4 of the hand piece 171 of the present embodiment and the second external display portion 153 of the handle unit 4 of the hand piece 151 of the third embodiment are formed in different patterns.

In the present embodiment, the configuration of the handle unit 4 is different from that of the handle unit 4 of the third embodiment. More specifically, in the handle unit 4 according to the present embodiment, a plural-fingers insertion ring section 177 of the fixed handle 176 is located below the holding tube 21 and extended to the proximal end side. At a proximal end portion of the plural-fingers insertion ring section 177, a nail-shaped finger placing portion 178 is provided in a protruding manner.

A movable handle 179 is extended above the holding tube 21. At one end portion of a movable handle 179, a protruding portion 180 substantially in a triangular shape is provided in a protruding manner.

It should be noted that the present invention is not limited to the embodiments described above. For example, the surgical apparatus may be a high-frequency surgical apparatus. The high-frequency surgical apparatus includes an electrical connection section configured to electrically connect a hand piece and a generator, the generator being configured to generate drive energy of driving the hand piece, and a conductive probe provided in the hand piece and electrically connected to the generator. A switch configured to control a high-frequency current supplied from the generator to the probe is made of the switch having the same configuration as the first switch 82 and the second switch 83 of the surgical operating apparatus according to the first embodiment (see FIGS. 1 to 15).

It is to be understood that the present invention can be modified and carried out in various manners without deviating from the gist of the present invention.

## Claims

1. An operation device (4) comprising:
an operation portion housing (61; 122) inside of which a cavity is formed, the cavity being opened with respect to an outside of the operation portion housing (61; 122) at an opening, the operation portion housing (61; 122) including a substrate holding section (95; 134) which forms a bottom surface of the cavity;
a switch substrate (86; 131) which is arranged on the substrate holding section (95; 134) in the cavity, the switch substrate (86; 131) including a switch contact point (90, 91), a common contact point (92, 93), and an urging member (94) configured to urge so that the switch contact point (90, 91) and the common contact point (92, 93) are not in contact with respect to each other;
a switch operation portion (87; 132) extending from the outside of the operation portion housing (61; 122) toward the cavity through the opening, and which is inclinable from a neutral position, the switch operation portion (87; 132) being straightly extended perpendicular to the bottom surface of the cavity in the neutral position, the switch operation portion (87; 132) including:
a pressure-receiving portion (97; 136) which is located to the outside of the operation portion housing (61; 122), and which is configured to receive a pressure toward the switch substrate (86; 131) in an operation;
a switch contact section (104; 142) which is located in the cavity, the switch contact section (104; 142) being configured to press the urging member (94) against an urging when the switch operation portion (87; 132) straightly moves toward the switch substrate (86, 131) from the neutral position by acting the pressure on the pressure-receiving portion (97; 136), and thereby the switch contact point (90, 91) and the common contact point (92, 93) being brought into contact against the urging of the urging member (94); and
a shaft section (98; 137) which is extended from the pressure-receiving portion (97; 136) toward the switch contact section (104; 142) through the opening;
wherein the operation device (4) further comprises a support portion (88; 135) configured to support the pressure-receiving portion (97) in such a manner that the pressure-receiving portion (97) is capable of moving about an inclination movement center (O) in the operation portion housing (61; 122) to incline with respect to the operation portion housing (61; 122),
the switch operation portion (87; 132) includes a protrusion (102, 103; 141) which is located in the cavity, and which protrudes from the shaft section (98; 137) toward an outer peripheral side of the shaft section (98; 137),
the operation portion housing (61; 122) includes an inner surface with which the protrusion (102, 103; 141) is configured to abut in the cavity when the switch operation portion (87; 132) inclines from the neutral position by acting the pressure on the pressure-receiving portion (97; 136),
the protrusion (102, 103; 141) being configured to abut the inner surface of the operation portion housing (61; 122) at a position located away from the shaft section (98; 137) toward a direction opposite to an inclination movement direction of the pressure-receiving portion (97; 136) and an inclination movement support point (105, 106) being formed at a contact section between the protrusion (102, 103; 141) and the inner surface of the operation portion housing (61; 122) during an inclination of the switch operation portion (87; 132) from the neutral position,
the switch contact section (104; 142) being configured to press the urging member (94) against an urging when the switch operation portion (87; 132) inclines about the inclination movement support point (105, 106), and thereby the switch contact point (90, 91) and the common contact point (92, 93) being brought into contact against the urging of the urging member (94), **characterized in that**
the support portion (88; 135) includes a clearance formation section (101; 140) configured to form a clearance between the support portion (88; 135) and a portion of the shaft section (98; 137), whereby the pressure-receiving portion (97; 136) moves about the inclination movement center (O), the clearance formation section (101; 140) including an inclination surface (101a; 140a) whose size of cross sectional area of the clearance gradually increases from the switch contact point (90, 91) side to the pressure-receiving portion (97; 136) side.

2. The operation device (4) according to claim 1 **characterized in that** the operation portion housing (61) includes a restricting section configured to restrict inclination directions of the switch operation portion (87) from the neutral position so that the pressure-receiving portion (97) is inclinable from the neutral position of the switch operation portion (87) toward only two inclination movement directions, the two inclination movement directions being a first inclination movement direction and a second inclination movement direction opposite to the first inclination movement direction.

3. The operation device (4) according to claim 2, **characterized in that** the protrusion (102, 103) includes:
a first protrusion (102) which protrudes from the shaft section (98) toward the first inclination movement direction of the pressure-receiving portion (97), the first protrusion (102) being configured to form a first inclination movement support point (105) at the contact section with the inner surface of the operation portion housing (61) when the pressure-receiving portion (97) of the switch operation portion (87) is inclined toward the second inclination movement direction from the neutral position; and
a second protrusion (103) which protrudes from the shaft section (98) toward the second inclination movement direction of the pressure-receiving portion (97), the second protrusion (103) being configured to form a second inclination movement support point (106) at the contact section with the inner surface of the operation portion housing (61) when the pressure-receiving portion (97) of the switch operation portion (87) is inclined toward the first inclination movement direction from the neutral position.

4. The operation device according to claim 2, **characterized in that**
the operation portion housing (61) includes a fixed handle (20) to which the switch operation portion (87) is attached, and a movable handle (22) which is openable and closable with respect to the fixed handle (20).

5. A surgical treatment apparatus **characterized by** comprising:
the operation device (4) according to one of claims 1 to 4;
a drive unit (8; 112, 113) which is configured to receive a driving signal which is transmitted from the switch substrate (86; 131) when the switch contact point (90, 91) and the common contact point (92, 93) are brought into contact, and which is configured to be driven based on a transmission of the driving signal; and
a treatment section (1A; 126) which is configured to receive at least one of an ultrasonic vibration and a high-frequency current which is transmitted when the drive unit (8; 112, 113) is driven.

## Patentansprüche

1. Betätigungsgerät (4), das umfasst:
ein Betätigungsabschnittsgehäuse (61; 122), in dessen Inneren ein Hohlraum gebildet ist, wobei der Hohlraum bezüglich einer Außenseite des Betätigungsabschnittsgehäuses (61; 122) an einer Öffnung geöffnet ist, wobei das Betätigungsabschnittsgehäuse (61; 122) einen Substrathalteabschnitt (95; 134) umfasst, der die Bodenfläche des Hohlraums bildet;
ein Schaltsubstrat (86; 131), das auf dem Substrathalteabschnitt (95; 134) in dem Hohlraum angeordnet ist, wobei das Schaltsubstrat (86; 131) einen Schaltkontaktpunkt (90, 91), einen gemeinsamen Kontaktpunkt (92, 93) und ein Vorspannelement (94) umfasst, das dazu eingerichtet ist, vorzuspannen, so dass der Schaltkontaktpunkt (90, 91) und der gemeinsame Kontaktpunkt (92, 93) nicht in Kontakt miteinander stehen;
einen Schaltbetätigungsabschnitt (87; 132), der sich von der Außenseite des Betätigungsabschnittsgehäuses (61; 122) in Richtung des Hohlraums durch die Öffnung erstreckt und der aus einer neutralen Position neigbar ist, wobei sich der Schaltbetätigungsabschnitt (87; 132) in der neutralen Position senkrecht zu der Bodenfläche des Hohlraums gerade erstreckt, wobei der Schaltbetätigungsabschnitt (87; 132) umfasst:
einen Druckempfangsabschnitt (97; 136), der an der Außenseite des Betätigungsabschnittsgehäuses (61; 122) angeordnet ist und der dazu eingerichtet ist, bei einer Betätigung einen Druck in Richtung des Schaltsubstrats (86; 131) zu empfangen;
einen Schaltkontaktabschnitt (104; 142), der in dem Hohlraum angeordnet ist, wobei der Schaltkontaktabschnitt (104; 142) dazu eingerichtet ist, das Vorspannelement (94) gegen eine Vorspannung zu drücken, wenn sich der Schaltbetätigungsabschnitt (87; 132) aus der neutralen Position gerade in Richtung des Schaltsubstrats (86, 131) bewegt, indem der Druck auf den Druckempfangsabschnitt (97; 136) aufgebracht wird und dadurch der Schaltkontaktpunkt (90, 91) und der gemeinsame Kontaktpunkt (92, 93) entgegen der Vorspannung des Vorspannelements (94) miteinander in Kontakt gebracht werden; und
einen Wellenabschnitt (98; 137), der sich von dem Druckempfangsabschnitt (97; 136) in Richtung des Schaltkontaktabschnitts (104; 142) durch die Öffnung erstreckt;
wobei das Betätigungsgerät (4) ferner einen Stützabschnitt (88; 135) umfasst, der dazu eingerichtet ist, den Druckempfangsabschnitt (97) derart zu abzustützen, dass der Druckempfangsabschnitt (97) dazu in der Lage ist, sich um ein Neigungsbewegungszentrum (O) in dem Betätigungsabschnittsgehäuse (61; 122) zu bewegen, um sich bezüglich des Betätigungsabschnittsgehäuses (61; 122) zu neigen,
der Schaltbetätigungsabschnitt (87; 132) einen Vorsprung (102, 103; 141) umfasst, der in dem Hohlraum angeordnet ist und der von dem Wellenabschnitt (98; 137) in Richtung einer Außenumfangsseite des Wellenabschnitts (98; 137) vorsteht,
das Betätigungsabschnittsgehäuse (61; 122) eine Innenfläche umfasst, an der der Vorsprung (102, 103; 141) in dem Hohlraum anzustoßen vermag, wenn sich der Schaltbetätigungsabschnitt (87; 132) aus der neutralen Position neigt, indem der Druck auf den Druckempfangsabschnitt (97; 136) aufgebracht wird,
der Vorsprung (102, 103; 141) dazu eingerichtet ist, an die Innenfläche des Betätigungsabschnittsgehäuses (61; 122) an einer Position anzustoßen, die von dem Wellenabschnitt (98; 137) in einer einer Neigungsbewegungsrichtung des Druckempfangsabschnitts (97; 136) entgegengesetzten Richtung entfernt angeordnet ist, und ein Neigungsbewegungsstützpunkt (105, 106) während einer Neigung des Schaltbetätigungsabschnitts (87; 132) aus der neutralen Position an einem Kontaktabschnitt zwischen dem Vorsprung (102, 103; 141) und der Innenfläche des Betätigungsabschnittsgehäuses (61; 122) ausgebildet ist,
der Schaltbetätigungsabschnitt (104; 142) dazu eingerichtet ist, das Vorspannelement (94) gegen eine Vorspannung zu drücken, wenn sich der Schaltbetätigungsabschnitt (87; 132) um den Neigungsbewegungsstützpunkt (105, 106) neigt und dadurch der Schaltkontaktpunkt (90, 91) und der gemeinsame Kontaktpunkt (92, 93) gegen die Vorspannung des Vorspannelements (94) in Kontakt miteinander gebracht werden,
**dadurch gekennzeichnet, dass**
der Stützabschnitt (88; 135) einen Spaltbildungsabschnitt (101; 140) umfasst, der dazu eingerichtet ist, einen Spalt zwischen dem Stützabschnitt (88; 135) und einem Abschnitt des Wellenabschnitts (98; 137) zu bilden, wodurch sich der Druckempfangsabschnitt (97; 136) um das Neigungsbewegungszentrum (O) bewegt, wobei der Spaltbildungsabschnitt (101; 140) eine Neigungsoberfläche (101a; 140a) umfasst, deren Querschnittsflächengröße des Spalts graduell von der Seite des Schaltkontaktpunkts (90, 91) zu der Seite des Druckempfangsabschnitts (97; 136) zunimmt.

2. Betätigungsgerät (4) gemäß Anspruch 1, **dadurch gekennzeichnet, dass** das Betätigungsabschnittsgehäuse (61) einen Begrenzungsabschnitt umfasst, der dazu eingerichtet ist, Neigungsrichtungen des Schaltbetätigungsabschnitts (87) aus der neutralen Position zu begrenzen, so dass der Druckempfangsabschnitt (97) aus der neutralen Position des Schaltbetätigungsabschnitts (87) nur in zwei Neigungsbewegungsrichtungen neigbar ist, wobei die zwei Neigungsbewegungsrichtungen eine erste Neigungsbewegungsrichtung und eine der ersten Neigungsbewegungsrichtung entgegengesetzte zweite Neigungsbewegungsrichtung sind.

3. Betätigungsgerät (4) gemäß Anspruch 2, **dadurch gekennzeichnet, dass** der Vorsprung (102, 103) umfasst:
einen ersten Vorsprung (102), der von dem Wellenabschnitt (98) in Richtung der ersten Neigungsbewegungsrichtung des Druckempfangsabschnitts (97) vorsteht, wobei der erste Vorsprung (102) dazu eingerichtet ist, einen ersten Neigungsbewegungsstützpunkt (105) an dem Kontaktabschnitt mit der Innenfläche des Betätigungsabschnittsgehäuses (61) zu bilden, wenn der Druckempfangsabschnitt (97) des Schaltbetätigungsabschnitts (87) aus der neutralen Position in die zweite Neigungsbewegungsrichtung geneigt wird; und
einen zweiten Vorsprung (103), der von dem Wellenabschnitt (98) in Richtung der zweiten Neigungsbewegungsrichtung des Druckempfangsabschnitts (97) vorsteht, wobei der zweite Vorsprung (103) dazu eingerichtet ist, einen zweiten Neigungsbewegungsstützpunkt (106) an dem Kontaktabschnitt mit der Innenfläche des Betätigungsabschnittsgehäuses (61) zu bilden, wenn der Druckempfangsabschnitt (97) des Schaltbetätigungsabschnitts (87) aus der neutralen Position in die erste Neigungsbewegungsrichtung geneigt wird.

4. Betätigungsgerät gemäß Anspruch 2, **dadurch gekennzeichnet, dass**
das Betätigungsabschnittsgehäuse (61) einen festen Griff (20), an dem der Schaltbetätigungsabschnitt (87) befestigt ist, und einen bewegbaren Griff (22) umfasst, der bezüglich des festen Griffs (20) geöffnet oder geschlossen zu werden vermag.

5. Chirurgisches Behandlungsgerät, **dadurch gekennzeichnet, dass** es umfasst:
das Betätigungsgerät (4) nach einem der Ansprüche 1 bis 4;
eine Antriebseinheit (8; 112, 113), die dazu eingerichtet ist, ein Antriebssignal zu empfangen, das von dem Schaltsubstrat (86; 131) übertragen wird, wenn der Schaltkontaktpunkt (90, 91) und der gemeinsame Kontaktpunkt (92, 93) miteinander in Kontakt gebracht werden, und das dazu eingerichtet ist, auf der Basis einer Übertragung des Antriebssignals angetrieben zu werden; und
ein Behandlungsabschnitt (1A; 126), der dazu eingerichtet ist, eine Ultraschallschwingung und/oder einen Hochfrequenzstrom zu empfangen, die/der übertragen wird, wenn die Antriebseinheit (8; 112, 113) angetrieben wird.

## Revendications

1. Dispositif d'actionnement (4) comprenant :
un logement (61 ; 122) de partie d'actionnement à l'intérieur duquel une cavité est formée, la cavité étant ouverte par rapport à l'extérieur du logement (61 ; 122) de partie d'actionnement au niveau d'une ouverture, le logement (61 ; 122) de partie d'actionnement incluant une section (95 ; 134) de maintien de substrat qui forme une surface de fond de la cavité ;
un substrat (86 ; 131) de commutateur qui est agencé sur la section (95 ; 134) de maintien de substrat dans la cavité, le substrat (86 ; 131) de commutateur incluant un point de contact de commutateur (90, 91), un point de contact commun (92, 93) et un élément de sollicitation (94) configuré pour solliciter de sorte que le point de contact de commutateur (98, 91) et le point de contact commun (92, 93) ne sont pas en contact l'un avec l'autre ;
une partie d'actionnement de commutateur (87 ; 132) s'étendant de l'extérieur du logement (61 ; 122) de partie d'actionnement vers la cavité à travers l'ouverture, et qui est inclinable par rapport à une position neutre, la partie d'actionnement de commutateur (87 ; 132) s'étendant de manière rectiligne perpendiculaire à la surface de fond de la cavité dans la position neutre, la partie d'actionnement de commutateur (87 ; 132) comprenant :
une partie de réception de pression (97 ; 136) qui est placée à l'extérieur du logement (61 ; 122) de partie d'actionnement et qui est configurée pour recevoir une pression vers le substrat (86 ; 131) de commutateur en fonctionnement ;
une section de contact de commutateur (104 ; 142) qui est placée dans la cavité, la section de contact de commutateur (104 ; 142) étant configurée pour presser l'élément de sollicitation (94) contre une sollicitation lorsque la partie d'actionnement de commutateur (87 ; 132) se déplace de manière rectiligne vers le substrat (86 ; 131) de commutateur depuis la position neutre en actionnant la pression sur la partie de réception de pression (97 ; 136) et de ce fait le point de contact de commutateur (90, 91) et le point de contact commun (92, 93) étant amenés en contact contre la sollicitation de l'élément de sollicitation (94) ; et
une section d'arbre (98 ; 137) qui s'étend depuis la partie de réception de pression (97 ; 136) vers la section de contact de commutateur (104 ; 142) à travers l'ouverture ;
dans lequel le dispositif d'actionnement (4) comprend en outre une partie de support (88 ; 135) configurée pour supporter la partie de réception de pression (97) d'une manière telle que la partie de réception de pression (97) est capable de se déplacer autour d'un centre de mouvement d'inclinaison (O) dans le logement (61 ; 122) de partie d'actionnement pour s'incliner par rapport au logement (61 ; 122) de partie d'actionnement,
la partie d'actionnement de commutateur (87 ; 132) comprend une saillie (102, 103 ; 141) qui est placée dans la cavité, et qui fait saillie depuis la section d'arbre (98 ; 137) vers un côté périphérique externe de la section d'arbre (98 ; 137),
le logement (61 ; 122) de partie d'actionnement comprend une surface interne avec laquelle la saillie (102, 103 ; 141) est configurée pour buter dans la cavité lorsque la partie d'actionnement de commutateur (87 ; 132) s'incline par rapport à la position neutre en actionnant la pression sur la partie de réception de pression (97 ; 136),
la saillie (102, 103 ; 141) étant configurée pour buter contre la surface interne du logement (61 ; 122) de partie d'actionnement au niveau d'une position placée éloignée de la section d'arbre (98 ; 137) vers une direction opposée à une direction de mouvement d'inclinaison de la partie de réception de pression (97 ; 136) et un point de support de mouvement d'inclinaison (105, 106) étant formé au niveau d'une section de contact entre la saillie (102, 103 ; 141) et la surface interne du logement (61 ; 122) de partie d'actionnement pendant une inclinaison de la partie d'actionnement de commutateur (87 ; 132) par rapport à la position neutre,
la section de contact de commutateur (104 ; 142) étant configurée pour presser l'élément de sollicitation (94) contre une sollicitation lorsque la partie d'actionnement de commutateur (87 ; 132) s'incline autour du point de support de mouvement d'inclinaison (105, 106), et de se fait le point de contact de commutateur (90, 91) et le point de contact commun (92, 93) étant amenés en contact contre la sollicitation de l'élément de sollicitation (94),
**caractérisé en ce que**
la partie de support (88 ; 135) comprend une section de formation de jeu (101 ; 140) configurée pour former un jeu entre la partie de support (88 ; 135) et une partie de la section d'arbre (98 ; 137), d'où il résulte que la partie de réception de pression (97 ; 136) se déplace autour du centre de mouvement d'inclinaison (O), la section de formation de jeu (101 ; 140) incluant une surface d'inclinaison (101a ; 140a) dont la taille de la zone en section transversale du jeu augmente progressivement en allant du côté point de contact de commutateur (90, 91) au côté partie de réception de pression (97 ; 136).

2. Dispositif d'actionnement (4) selon la revendication 1, **caractérisé en ce que** le logement (61) de partie d'actionnement comprend une section de limitation configurée pour limiter les directions d'inclinaison de la partie d'actionnement de commutateur (87) depuis la position neutre de sorte que la partie de réception de pression (97) est inclinable depuis la position neutre de la partie d'actionnement de commutateur (87) uniquement vers deux directions de mouvement d'inclinaison, les deux directions de mouvement d'inclinaison correspondant à une première direction de mouvement d'inclinaison et une deuxième direction de mouvement d'inclinaison opposée à la première direction de mouvement d'inclinaison.

3. Dispositif d'actionnement (4) selon la revendication 2, **caractérisé en ce que** la saillie (102, 103) comprend :
une première saillie (102) qui fait saillie depuis la section d'arbre (98) vers la première direction de mouvement d'inclinaison de la partie de réception de pression (97), la première saillie (102) étant configurée pour former un premier point de support de mouvement d'inclinaison (105) au niveau de la section de contact avec la surface interne du logement (61) de partie d'actionnement lorsque la partie de réception de pression (97) de la partie d'actionnement de commutateur (87) est inclinée vers la deuxième direction de mouvement d'inclinaison depuis la position neutre ; et
une deuxième saillie (103) qui fait saillie depuis la section d'arbre (98) vers la deuxième direction de mouvement d'inclinaison de la partie de réception de pression (97), la deuxième saillie (103) étant configurée pour former un deuxième point de support de mouvement d'inclinaison (106) au niveau de la section de contact avec la surface interne du logement (61) de partie d'actionnement lorsque la partie de réception de pression (97) de la partie d'actionnement de commutateur (87) est inclinée vers la première direction de mouvement d'inclinaison depuis la position neutre.

4. Dispositif d'actionnement selon la revendication 2, **caractérisé en ce que**
le logement (61) de partie d'actionnement comprend une poignée fixe (20) à laquelle la partie d'actionnement de commutateur (87) est attachée, et une poignée mobile (22) qui peut être ouverte et fermée par rapport à la poignée fixe (20).

5. Appareil de traitement chirurgical **caractérisé en ce qu'**il comprend :
le dispositif d'actionnement (4) selon l'une quelconque des revendications 1 à 4 ;
une unité d'entraînement (8 ; 112, 113) qui est configurée pour recevoir un signal d'entraînement qui est transmis depuis le substrat (86 ; 131) de commutateur lorsque le point de contact de commutateur (90, 91) et le point de contact commun (92, 93) sont amenés en contact, et qui est configurée pour être entraînée sur la base d'une transmission du signal d'entraînement ; et
une section de traitement (1A ; 126) qui est configurée pour recevoir au moins l'un parmi une vibration ultrasonore et un courant haute fréquence qui est transmis lorsque l'unité d'entraînement (8 ; 112, 113) est entraînée.
